# EUROPÄISCHE PATENTANMELDUNG

(11) **EP 2 098 592 A2**
(43) Veröffentlichungstag der Anmeldung: **09.09.2009**
(21) Anmeldenummer: 09155984.9
(22) Anmeldetag: 25.07.2006
(51) Int. Cl.: C12N 9/12, C12Q 1/68

(54) **RNA-abhängige RNA-Polymerase, Verfahren und Kits zur Amplifikation und/oder Markierung von RNA**

(30) Priorität: 25.07.2005 DE 102005036085; 13.02.2006 DE 102006008219
(62) Teilanmeldung aus: 06761834.8
(71) Anmelder: Technische Universität Dresden, 01062 Dresden (DE)
(72) Erfinder: Rohayem, Jacques, 01309, Dresden (DE); Jäger, Katrin, 01462, Dresden (DE); Jacobs, Enno, 01309, Dresden (DE); Rudolph, Wolfram, 01307, Dresden (DE)
(74) Vertreter: Habermann, Gert

(57) **Zusammenfassung**

Die vorliegende Erfindung betrifft eine RNA-abhängige RNA-Polymerase eines Sapovirus sowie Kits zur Amplifikation und Markierung von RNA, welche die RNA-abhängige RNA-Polymerase enthalten.

SEQUENCE LISTING

<110>  Technische Universität Dresden

<120>  RNA-abhängige RNA-Polymerase, Verfahren und Kits zur
       Amplifikation und/oder Markierung von RNA

<130>  T 0030 WOEPT2

<150>  DE 10 2005 036 085.8
<151>  2005-07-25

<150>  DE 10 2006 008 219.2
<151>  2006-02-13

<160>  6

<170>  PatentIn version 3.3

<210>  1
<211>  520
<212>  PRT
<213>  Norovirus

<400>  1

Met Gly Gly Asp Ser Lys Gly Thr Tyr Cys Gly Ala Pro Ile Leu Gly
1               5                   10                  15

Pro Gly Ser Ala Pro Lys Leu Ser Thr Lys Thr Lys Phe Trp Arg Ser
            20                  25                  30

Ser Thr Thr Pro Leu Pro Pro Gly Thr Tyr Glu Pro Ala Tyr Leu Gly
        35                  40                  45

Gly Lys Asp Pro Arg Val Lys Gly Gly Pro Ser Leu Gln Gln Val Met
    50                  55                  60

Arg Asp Gln Leu Lys Pro Phe Thr Glu Pro Arg Gly Lys Pro Pro Lys
65                  70                  75                  80

Pro Ser Val Leu Glu Ala Ala Lys Lys Thr Ile Ile Asn Val Leu Glu
                85                  90                  95

Gln Thr Ile Asp Pro Pro Glu Lys Trp Ser Phe Thr Gln Ala Cys Ala
            100                 105                 110

Ser Leu Asp Lys Thr Thr Ser Ser Gly His Pro His His Met Arg Lys
        115                 120                 125

Asn Asp Cys Trp Asn Gly Glu Ser Phe Thr Gly Lys Leu Ala Asp Gln
    130                 135                 140

Ala Ser Lys Ala Asn Leu Met Phe Glu Gly Gly Lys Asn Met Thr Pro
145                 150                 155                 160

Val Tyr Thr Gly Ala Leu Lys Asp Glu Leu Val Lys Thr Asp Lys Ile
                165                 170                 175

Tyr Gly Lys Ile Lys Lys Arg Leu Leu Trp Gly Ser Asp Leu Ala Thr
            180                 185                 190

Met Ile Arg Cys Ala Arg Ala Phe Gly Gly Leu Met Asp Glu Leu Lys
        195                 200                 205

Ala His Cys Val Thr Leu Pro Ile Arg Val Gly Met Asn Met Asn Glu
    210                 215                 220

Asp Gly Pro Ile Ile Phe Glu Arg His Ser Arg Tyr Lys Tyr His Tyr
225                 230                 235                 240

Asp Ala Asp Tyr Ser Arg Trp Asp Ser Thr Gln Gln Arg Ala Val Leu
                245                 250                 255

Ala Ala Ala Leu Glu Ile Met Val Lys Phe Ser Ser Glu Pro His Leu
            260                 265                 270

Ala Gln Val Val Ala Glu Asp Leu Leu Ser Pro Ser Val Val Asp Val
        275                 280                 285

Gly Asp Phe Lys Ile Ser Ile Asn Glu Gly Leu Pro Ser Gly Val Pro
    290                 295                 300

Cys Thr Ser Gln Trp Asn Ser Ile Ala His Trp Leu Leu Thr Leu Cys
305                 310                 315                 320

Ala Leu Ser Glu Val Thr Asn Leu Ser Pro Asp Ile Ile Gln Ala Asn
                325                 330                 335

Ser Leu Phe Ser Phe Tyr Gly Asp Asp Glu Ile Val Ser Thr Asp Ile
            340                 345                 350

Lys Leu Asp Pro Glu Lys Leu Thr Ala Lys Leu Lys Glu Tyr Gly Leu
        355                 360                 365

Lys Pro Thr Arg Pro Asp Lys Thr Glu Gly Pro Leu Val Ile Ser Glu
    370                 375                 380

Asp Leu Asn Gly Leu Thr Phe Leu Arg Arg Thr Val Thr Arg Asp Pro
385                 390                 395                 400

Ala Gly Trp Phe Gly Lys Leu Glu Gln Ser Ser Ile Leu Arg Gln Met
                405                 410                 415

Tyr Trp Thr Arg Gly Pro Asn His Glu Asp Pro Ser Glu Thr Met Ile
            420                 425                 430

Pro His Ser Gln Arg Pro Ile Gln Leu Met Ser Leu Leu Gly Glu Ala
        435                 440                 445

Ala Leu His Gly Pro Ala Phe Tyr Ser Lys Ile Ser Lys Leu Val Ile
    450                 455                 460

Ala Glu Leu Lys Glu Gly Gly Met Asp Phe Tyr Val Pro Arg Gln Glu
465                 470                 475                 480

Pro Met Phe Arg Trp Met Arg Phe Ser Asp Leu Ser Thr Trp Glu Gly
                485                 490                 495

Asp Arg Asn Leu Ala Pro Ser Phe Val Asn Glu Asp Gly Val Glu Val
            500                 505                 510

Asp Lys Leu Ala Ala Ala Leu Glu
        515                 520

<210>  2
<211>  517
<212>  PRT
<213>  Sapovirus

<400>  2

Met Lys Asp Glu Phe Gln Trp Lys Gly Leu Pro Val Val Lys Ser Gly
1               5                   10                  15

Leu Asp Val Gly Gly Met Pro Thr Gly Thr Arg Tyr His Arg Ser Pro
            20                  25                  30

Ala Trp Pro Glu Glu Gln Pro Gly Glu Thr His Ala Pro Ala Pro Phe
        35                  40                  45

Gly Ala Gly Asp Lys Arg Tyr Thr Phe Ser Gln Thr Glu Met Leu Val
    50                  55                  60

Asn Gly Leu Lys Pro Tyr Thr Glu Pro Thr Ala Gly Val Pro Pro Gln
65                  70                  75                  80

Leu Leu Ser Arg Ala Val Thr His Val Arg Ser Tyr Ile Glu Thr Ile
                85                  90                  95

Ile Gly Thr His Arg Ser Pro Val Leu Thr Tyr His Gln Ala Cys Glu
            100                 105                 110

Leu Leu Glu Arg Thr Thr Ser Cys Gly Pro Phe Val Gln Gly Leu Lys
        115                 120                 125

Gly Asp Tyr Trp Asp Glu Glu Gln Gln Gln Tyr Thr Gly Val Leu Ala
    130                 135                 140

Asn His Leu Glu Gln Ala Trp Asp Lys Ala Asn Lys Gly Ile Ala Pro
145                 150                 155                 160

Arg Asn Ala Tyr Lys Leu Ala Leu Lys Asp Glu Leu Arg Pro Ile Glu
                165                 170                 175

Lys Asn Lys Ala Gly Lys Arg Arg Leu Leu Trp Gly Cys Asp Ala Ala
            180                 185                 190

Thr Thr Leu Ile Ala Thr Ala Ala Phe Lys Ala Val Ala Thr Arg Leu
        195                 200                 205

Gln Val Val Thr Pro Met Thr Pro Val Ala Val Gly Ile Asn Met Asp
    210                 215                 220

Ser Val Gln Met Gln Val Met Asn Asp Ser Leu Lys Gly Gly Val Leu
225                 230                 235                 240

Tyr Cys Leu Asp Tyr Ser Lys Trp Asp Ser Thr Gln Asn Pro Ala Val
                245                 250                 255

Thr Ala Ala Ser Leu Ala Ile Leu Glu Arg Phe Ala Glu Pro His Pro
            260                 265                 270

Ile Val Ser Cys Ala Ile Glu Ala Leu Ser Ser Pro Ala Glu Gly Tyr
        275                 280                 285

Val Asn Asp Ile Lys Phe Val Thr Arg Gly Gly Leu Pro Ser Gly Met
    290                 295                 300

Pro Phe Thr Ser Val Val Asn Ser Ile Asn His Met Ile Tyr Val Ala
305                 310                 315                 320

Ala Ala Ile Leu Gln Ala Tyr Glu Ser His Asn Val Pro Tyr Thr Gly
                325                 330                 335

Asn Val Phe Gln Val Glu Thr Val His Thr Tyr Gly Asp Asp Cys Met
            340                 345                 350

Tyr Ser Val Cys Pro Ala Thr Ala Ser Ile Phe His Ala Val Leu Ala
        355                 360                 365

Asn Leu Thr Ser Tyr Gly Leu Lys Pro Thr Ala Ala Asp Lys Ser Asp
    370                 375                 380

Ala Ile Lys Pro Thr Asn Thr Pro Val Phe Leu Lys Arg Thr Phe Thr
385                 390                 395                 400

Gln Thr Pro His Gly Val Arg Ala Leu Leu Asp Ile Thr Ser Ile Thr
                405                 410                 415

Arg Gln Phe Tyr Trp Leu Lys Ala Asn Arg Thr Ser Asp Pro Ser Ser
            420                 425                 430

Pro Pro Ala Phe Asp Arg Gln Ala Arg Ser Ala Gln Leu Glu Asn Ala
        435                 440                 445

Leu Ala Tyr Ala Ser Gln His Gly Pro Val Val Phe Asp Thr Val Arg
    450                 455                 460

Gln Ile Ala Ile Lys Thr Ala Gln Gly Glu Gly Leu Val Leu Val Asn
465                 470                 475                 480

Thr Asn Tyr Asp Gln Ala Leu Ala Thr Tyr Asn Ala Trp Phe Ile Gly
                485                 490                 495

Gly Thr Val Pro Asp Pro Val Gly His Thr Glu Gly Thr His Lys Ile
            500                 505                 510

Val Phe Glu Met Glu
        515

<210>  3
<211>  533
<212>  PRT
<213>  Vesivirus

<400>  3
   Met Lys Val Thr Thr Gln Lys Tyr Asp Val Thr Lys Pro Asp Ile Ser
   1               5                   10                  15
   Tyr Lys Gly Leu Ile Cys Lys Gln Leu Asp Glu Ile Arg Val Ile Pro
               20                  25                  30
   Lys Gly Thr Arg Leu His Val Ser Pro Ala His Thr Asp Asp Tyr Asp
           35                  40                  45
   Glu Cys Ser His Gln Pro Ala Ser Leu Gly Ser Gly Asp Pro Arg Cys
       50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145                 150                 155                 160
   Ala Thr Gln Gly Ile Gly Leu Pro His Glu Tyr Thr Ile Gly Leu Lys
                   165                 170                 175
   Asp Glu Leu Arg Pro Val Glu Lys Val Gln Glu Gly Lys Arg Arg Met
               180                 185                 190
   Ile Trp Gly Cys Asp Val Gly Val Ala Thr Val Cys Ala Ala Ala Phe
           195                 200                 205
   Lys Gly Val Ser Asp Ala Ile Thr Ala Asn His Gln Tyr Gly Pro Val
       210                 215                 220
   Gln Val Gly Ile Asn Met Asp Gly Pro Ser Val Glu Ala Leu Tyr Gln
   225                 230                 235                 240
   Arg Ile Arg Ser Ala Ala Lys Val Phe Ala Val Asp Tyr Ser Lys Trp
                   245                 250                 255
   Asp Ser Thr Gln Ser Pro Arg Val Ser Ala Ala Ser Ile Asp Ile Leu
               260                 265                 270
   Arg Tyr Phe Ser Asp Arg Ser Pro Ile Val Asp Ser Ala Ala Asn Thr
           275                 280                 285
   Leu Lys Ser Pro Pro Ile Ala Ile Phe Asn Gly Val Ala Val Lys Val
       290                 295                 300
   Thr Ser Gly Leu Pro Ser Gly Met Pro Leu Thr Ser Val Ile Asn Ser
   305                 310                 315                 320
   Leu Asn His Cys Leu Tyr Val Gly Cys Ala Ile Leu Gln Ser Leu Glu
                   325                 330                 335
   Ser Arg Asn Ile Pro Val Thr Trp Asn Leu Phe Ser Thr Phe Asp Met
               340                 345                 350
   Met Thr Tyr Gly Asp Asp Gly Val Tyr Met Phe Pro Met Met Phe Ala
           355                 360                 365
   Ser Val Ser Asp Gln Ile Phe Ala Asn Leu Thr Ala Tyr Gly Leu Lys
       370                 375                 380
   Pro Thr Arg Val Asp Lys Ser Val Gly Ala Ile Glu Pro Ile Asp Pro
   385                 390                 395                 400
   Glu Ser Val Val Phe Leu Lys Arg Thr Ile Thr Arg Thr Pro His Gly
                   405                 410                 415
   Ile Arg Gly Leu Leu Asp Arg Gly Ser Ile Ile Arg Gln Phe Tyr Tyr
               420                 425                 430
   Ile Lys Gly Glu Asn Ser Asp Asp Trp Lys Thr Pro Pro Lys Thr Ile
           435                 440                 445
   Asp Pro Thr Ser Arg Gly Gln Gln Leu Trp Asn Ala Cys Leu Tyr Ala
       450                 455                 460
   Ser Gln His Gly Pro Glu Phe Tyr Asn Lys Val Tyr Arg Leu Ala Glu
   465                 470                 475                 480
   Lys Ala Val Glu Tyr Glu Glu Leu His Phe Glu Pro Pro Ser Tyr His
                   485                 490                 495
   Ser Ala Leu Glu His Tyr Asn Asn Gln Phe Asn Gly Val Asp Thr Arg
               500                 505                 510
   Ser Asp Gln Ile Asp Ala Ser Val Met Thr Asp Leu His Cys Asp Val
           515                 520                 525
   Phe Glu Val Leu Glu
       530

<210> 4
<211> 526
<212> PRT
<213> Artificial

<220>
<223> Norovirus-RdRP mit einem Hisitiden-Marker

<400> 4

Met Gly Gly Asp Ser Lys Gly Thr Tyr Cys Gly Ala Pro Ile Leu Gly
1               5                   10                  15

Pro Gly Ser Ala Pro Lys Leu Ser Thr Lys Thr Lys Phe Trp Arg Ser
            20                  25                  30

Ser Thr Thr Pro Leu Pro Pro Gly Thr Tyr Glu Pro Ala Tyr Leu Gly
        35                  40                  45

Gly Lys Asp Pro Arg Val Lys Gly Gly Pro Ser Leu Gln Gln Val Met
    50                  55                  60

Arg Asp Gln Leu Lys Pro Phe Thr Glu Pro Arg Gly Lys Pro Pro Lys
65                  70                  75                  80

Pro Ser Val Leu Glu Ala Ala Lys Lys Thr Ile Ile Asn Val Leu Glu
                85                  90                  95

Gln Thr Ile Asp Pro Pro Glu Lys Trp Ser Phe Thr Gln Ala Cys Ala
            100                 105                 110

Ser Leu Asp Lys Thr Thr Ser Ser Gly His Pro His His Met Arg Lys
        115                 120                 125

Asn Asp Cys Trp Asn Gly Glu Ser Phe Thr Gly Lys Leu Ala Asp Gln
    130                 135                 140

Ala Ser Lys Ala Asn Leu Met Phe Glu Gly Gly Lys Asn Met Thr Pro
145                 150                 155                 160

Val Tyr Thr Gly Ala Leu Lys Asp Glu Leu Val Lys Thr Asp Lys Ile
                165                 170                 175

Tyr Gly Lys Ile Lys Lys Arg Leu Leu Trp Gly Ser Asp Leu Ala Thr
            180                 185                 190

Met Ile Arg Cys Ala Arg Ala Phe Gly Gly Leu Met Asp Glu Leu Lys
        195                 200                 205

Ala His Cys Val Thr Leu Pro Ile Arg Val Gly Met Asn Met Asn Glu
    210                 215                 220

Asp Gly Pro Ile Ile Phe Glu Arg His Ser Arg Tyr Lys Tyr His Tyr
225                 230                 235                 240

Asp Ala Asp Tyr Ser Arg Trp Asp Ser Thr Gln Gln Arg Ala Val Leu
                245                 250                 255

Ala Ala Ala Leu Glu Ile Met Val Lys Phe Ser Ser Glu Pro His Leu
            260                 265                 270

Ala Gln Val Val Ala Glu Asp Leu Leu Ser Pro Ser Val Val Asp Val
        275                 280                 285

Gly Asp Phe Lys Ile Ser Ile Asn Glu Gly Leu Pro Ser Gly Val Pro
    290                 295                 300

Cys Thr Ser Gln Trp Asn Ser Ile Ala His Trp Leu Leu Thr Leu Cys
305                 310                 315                 320

Ala Leu Ser Glu Val Thr Asn Leu Ser Pro Asp Ile Ile Gln Ala Asn
                325                 330                 335

Ser Leu Phe Ser Phe Tyr Gly Asp Asp Glu Ile Val Ser Thr Asp Ile
            340                 345                 350

Lys Leu Asp Pro Glu Lys Leu Thr Ala Lys Leu Lys Glu Tyr Gly Leu
        355                 360                 365

Lys Pro Thr Arg Pro Asp Lys Thr Glu Gly Pro Leu Val Ile Ser Glu
    370                 375                 380

Asp Leu Asn Gly Leu Thr Phe Leu Arg Arg Thr Val Thr Arg Asp Pro
385                 390                 395                 400

Ala Gly Trp Phe Gly Lys Leu Glu Gln Ser Ser Ile Leu Arg Gln Met
                405                 410                 415

Tyr Trp Thr Arg Gly Pro Asn His Glu Asp Pro Ser Glu Thr Met Ile
            420                 425                 430

Pro His Ser Gln Arg Pro Ile Gln Leu Met Ser Leu Leu Gly Glu Ala
        435                 440                 445

Ala Leu His Gly Pro Ala Phe Tyr Ser Lys Ile Ser Lys Leu Val Ile
    450                 455                 460

Ala Glu Leu Lys Glu Gly Gly Met Asp Phe Tyr Val Pro Arg Gln Glu
465                 470                 475                 480

Pro Met Phe Arg Trp Met Arg Phe Ser Asp Leu Ser Thr Trp Glu Gly
                485                 490                 495

Asp Arg Asn Leu Ala Pro Ser Phe Val Asn Glu Asp Gly Val Glu Val
            500                 505                 510

Asp Lys Leu Ala Ala Ala Leu Glu His His His His His His
        515                 520                 525

<210>  5
<211>  523
<212>  PRT
<213>  Artificial

<220>
<223>  Sapovirus-RdRP mit einem Histidin-Marker

<400>  5

Met Lys Asp Glu Phe Gln Trp Lys Gly Leu Pro Val Val Lys Ser Gly
1               5                   10                  15

Leu Asp Val Gly Gly Met Pro Thr Gly Thr Arg Tyr His Arg Ser Pro
            20                  25                  30

Ala Trp Pro Glu Glu Gln Pro Gly Glu Thr His Ala Pro Ala Pro Phe
        35                  40                  45

Gly Ala Gly Asp Lys Arg Tyr Thr Phe Ser Gln Thr Glu Met Leu Val
    50                  55                  60

Asn Gly Leu Lys Pro Tyr Thr Glu Pro Thr Ala Gly Val Pro Pro Gln
65                  70                  75                  80

Leu Leu Ser Arg Ala Val Thr His Val Arg Ser Tyr Ile Glu Thr Ile
                85                  90                  95

Ile Gly Thr His Arg Ser Pro Val Leu Thr Tyr His Gln Ala Cys Glu
            100                 105                 110

Leu Leu Glu Arg Thr Thr Ser Cys Gly Pro Phe Val Gln Gly Leu Lys
        115                 120                 125

Gly Asp Tyr Trp Asp Glu Glu Gln Gln Gln Tyr Thr Gly Val Leu Ala
    130                 135                 140

Asn His Leu Glu Gln Ala Trp Asp Lys Ala Asn Lys Gly Ile Ala Pro
145                 150                 155                 160

Arg Asn Ala Tyr Lys Leu Ala Leu Lys Asp Glu Leu Arg Pro Ile Glu
                165                 170                 175

Lys Asn Lys Ala Gly Lys Arg Arg Leu Leu Trp Gly Cys Asp Ala Ala
            180                 185                 190

Thr Thr Leu Ile Ala Thr Ala Ala Phe Lys Ala Val Ala Thr Arg Leu
        195                 200                 205

Gln Val Val Thr Pro Met Thr Pro Val Ala Val Gly Ile Asn Met Asp
    210                 215                 220

Ser Val Gln Met Gln Val Met Asn Asp Ser Leu Lys Gly Gly Val Leu
225                 230                 235                 240

Tyr Cys Leu Asp Tyr Ser Lys Trp Asp Ser Thr Gln Asn Pro Ala Val
                245                 250                 255

Thr Ala Ala Ser Leu Ala Ile Leu Glu Arg Phe Ala Glu Pro His Pro
            260                 265                 270

Ile Val Ser Cys Ala Ile Glu Ala Leu Ser Ser Pro Ala Glu Gly Tyr
        275                 280                 285

Val Asn Asp Ile Lys Phe Val Thr Arg Gly Gly Leu Pro Ser Gly Met
    290                 295                 300

Pro Phe Thr Ser Val Val Asn Ser Ile Asn His Met Ile Tyr Val Ala
305                 310                 315                 320

Ala Ala Ile Leu Gln Ala Tyr Glu Ser His Asn Val Pro Tyr Thr Gly
                325                 330                 335

Asn Val Phe Gln Val Glu Thr Val His Thr Tyr Gly Asp Asp Cys Met
            340                 345                 350

Tyr Ser Val Cys Pro Ala Thr Ala Ser Ile Phe His Ala Val Leu Ala
        355                 360                 365

Asn Leu Thr Ser Tyr Gly Leu Lys Pro Thr Ala Ala Asp Lys Ser Asp
    370                 375                 380

Ala Ile Lys Pro Thr Asn Thr Pro Val Phe Leu Lys Arg Thr Phe Thr
385                 390                 395                 400

Gln Thr Pro His Gly Val Arg Ala Leu Leu Asp Ile Thr Ser Ile Thr
                405                 410                 415

Arg Gln Phe Tyr Trp Leu Lys Ala Asn Arg Thr Ser Asp Pro Ser Ser
            420                 425                 430

Pro Pro Ala Phe Asp Arg Gln Ala Arg Ser Ala Gln Leu Glu Asn Ala
        435                 440                 445

Leu Ala Tyr Ala Ser Gln His Gly Pro Val Val Phe Asp Thr Val Arg
    450                 455                 460

Gln Ile Ala Ile Lys Thr Ala Gln Gly Glu Gly Leu Val Leu Val Asn
465                 470                 475                 480

Thr Asn Tyr Asp Gln Ala Leu Ala Thr Tyr Asn Ala Trp Phe Ile Gly
                485                 490                 495

Gly Thr Val Pro Asp Pro Val Gly His Thr Glu Gly Thr His Lys Ile
            500                 505                 510

Val Phe Glu Met Glu His His His His His His
        515                 520

<210>  6
<211>  539
<212>  PRT
<213>  Artificial

<220>
<223>  Vesivirus-RdRP mit einem Histidin-Marker

<400>  6

Met Lys Val Thr Thr Gln Lys Tyr Asp Val Thr Lys Pro Asp Ile Ser
1               5                   10                  15

Tyr Lys Gly Leu Ile Cys Lys Gln Leu Asp Glu Ile Arg Val Ile Pro
            20                  25                  30

Lys Gly Thr Arg Leu His Val Ser Pro Ala His Thr Asp Asp Tyr Asp
        35                  40                  45

Glu Cys Ser His Gln Pro Ala Ser Leu Gly Ser Gly Asp Pro Arg Cys
    50                  55                  60

Pro Lys Ser Leu Thr Ala Ile Val Val Asp Ser Leu Lys Pro Tyr Cys
65                  70                  75                  80

Glu Lys Thr Asp Gly Pro Pro His Asp Ile Leu His Arg Val Gln Arg
                85                  90                  95

Met Leu Ile Asp His Leu Ser Gly Phe Val Pro Met Asn Ile Ser Ser
            100                 105                 110

Glu Pro Ser Met Leu Ala Ala Phe His Lys Leu Asn His Asp Thr Ser
        115                 120                 125

Cys Gly Pro Tyr Leu Gly Gly Arg Lys Lys Asp His Met Ile Gly Gly
    130                 135                 140

Glu Pro Asp Lys Pro Leu Leu Asp Leu Leu Ser Ser Lys Trp Lys Leu
145                 150                 155                 160

Ala Thr Gln Gly Ile Gly Leu Pro His Glu Tyr Thr Ile Gly Leu Lys
                165                 170                 175

Asp Glu Leu Arg Pro Val Glu Lys Val Gln Glu Gly Lys Arg Arg Met
            180                 185                 190

Ile Trp Gly Cys Asp Val Gly Val Ala Thr Val Cys Ala Ala Ala Phe
        195                 200                 205

Lys Gly Val Ser Asp Ala Ile Thr Ala Asn His Gln Tyr Gly Pro Val
    210                 215                 220

Gln Val Gly Ile Asn Met Asp Gly Pro Ser Val Glu Ala Leu Tyr Gln
225                 230                 235                 240

Arg Ile Arg Ser Ala Ala Lys Val Phe Ala Val Asp Tyr Ser Lys Trp
                245                 250                 255

Asp Ser Thr Gln Ser Pro Arg Val Ser Ala Ala Ser Ile Asp Ile Leu
            260                 265                 270

Arg Tyr Phe Ser Asp Arg Ser Pro Ile Val Asp Ser Ala Ala Asn Thr
        275                 280                 285

Leu Lys Ser Pro Pro Ile Ala Ile Phe Asn Gly Val Ala Val Lys Val
    290                 295                 300

Thr Ser Gly Leu Pro Ser Gly Met Pro Leu Thr Ser Val Ile Asn Ser
305                 310                 315                 320

Leu Asn His Cys Leu Tyr Val Gly Cys Ala Ile Leu Gln Ser Leu Glu
                325                 330                 335

Ser Arg Asn Ile Pro Val Thr Trp Asn Leu Phe Ser Thr Phe Asp Met
            340                 345                 350

Met Thr Tyr Gly Asp Asp Gly Val Tyr Met Phe Pro Met Met Phe Ala
        355                 360                 365

Ser Val Ser Asp Gln Ile Phe Ala Asn Leu Thr Ala Tyr Gly Leu Lys
    370                 375                 380

Pro Thr Arg Val Asp Lys Ser Val Gly Ala Ile Glu Pro Ile Asp Pro
385                 390                 395                 400

Glu Ser Val Val Phe Leu Lys Arg Thr Ile Thr Arg Thr Pro His Gly
                405                 410                 415

Ile Arg Gly Leu Leu Asp Arg Gly Ser Ile Ile Arg Gln Phe Tyr Tyr
            420                 425                 430

Ile Lys Gly Glu Asn Ser Asp Asp Trp Lys Thr Pro Pro Lys Thr Ile
        435                 440                 445

Asp Pro Thr Ser Arg Gly Gln Gln Leu Trp Asn Ala Cys Leu Tyr Ala
    450                 455                 460

Ser Gln His Gly Pro Glu Phe Tyr Asn Lys Val Tyr Arg Leu Ala Glu
465                 470                 475                 480

Lys Ala Val Glu Tyr Glu Glu Leu His Phe Glu Pro Pro Ser Tyr His
                485                 490                 495

Ser Ala Leu Glu His Tyr Asn Asn Gln Phe Asn Gly Val Asp Thr Arg
            500                 505                 510

Ser Asp Gln Ile Asp Ala Ser Val Met Thr Asp Leu His Cys Asp Val
        515                 520                 525

Phe Glu Val Leu Glu His His His His His His
    530                 535

## Beschreibung

Die Erfindung betrifft eine RNA-abhängige RNA-Polymerase sowie Verfahren und Kits zur Markierung und / oder zur primerabhängigen und -unabhängigen Amplifikation von Ribonukleinsäure (RNA), insbesondere von viraler, eukaryontischer und prokaryontischer und doppeisträngiger Ribonukleinsäure (RNA) zur Anwendung in der Biotechnologie und der Medizin. Das erfindungsgemäße Amplifikationsverfahren eignet sich insbesondere für die Microarray-Technologie, zur Herstellung von siRNA und zur Diagnose viraler Infektionen durch den Nachweis viraler RNA in Patientenmaterial. Das erfindungsgemäße Markierungsverfahren eignet sich insbesondere für die Aufreinigung von RNA mittels Affinitäts-Bindung, sowie für die Markierung von RNA für die Anwendung in molekularbiologischen Verfahren, die bei der Charakterisierung der Funktion und/oder Struktur viraler, eukaryontischer und prokaryontischer und doppelsträngiger Ribonukleinsäure (RNA) verwendet werden.

Die RNA ist ein wichtiger Bestandteil der eukaryontischen und der prokaryontischen Zelle. Sie ist an mehreren vitalen Funktionen beteiligt: der Transkription, d.h. der Umschreibung der Information des Erbgutes (Desoxyribonukleinsäure, DNA), der Übertragung dieser Information vom Zellkern in das Zytoplasma (mRNA), und der Translation, d.h. die Übersetzung der umgeschriebenen Information in Aminosäuren bzw. Proteine. Die RNA bildet auch die sog. Transfer-RNA, einen wichtigen Baustein für die Translation. Die RNA ist auch ein wichtiger Bestandteil der Ribosomen. Dies sind strukturelle Einheiten des Zytoplasmas, an denen die Übersetzung der Information in Eiweiß stattfindet.

Die Bedeutung der RNA im Bereich der Biotechnologie hat in den letzten Jahrzehnten zugenommen. Neue Entwicklungen wie z.B. die Microarray-Technologie, die miRNA (micro-RNA) oder die siRNA Technologie (small interfering RNA) haben stattgefunden, deren Relevanz im Bereich der Grundlagenforschung als auch der angewandten Forschung zunimmt. All diese Technologien beruhen auf der synthetischen Generierung von RNA durch Amplifikation *in vitro.*

Die Herstellung von siPNA *in vitro* erfolgt bislang über chemische Synthese oder mit Hilfe von DNA abhängigen RNA-Polymerasen wie der T7-RNA-Polyrmerase. Dafür werden zwei komplementäre DNA-Stränge mittels PCR unter Einsatz eines Primers, der auch die Sequenz für einen T7-Promotor enthält synthetisiert. Nach Umschreiben der DNA mit Hilfe der T7 RNA-Polymerase werden die resultierenden komplementären RNA-Stränge hybridisiert und mittels RNAse 1 verdaut.

Im medizinischen Bereich spielt die RNA-Amplifikation eine wichtige Rolle beim Nachweis viraler Infektionen im Patientenmaterial. Weltweit werden 80% aller viralen Infektionen durch RNA-Viren, d.h. virale Erreger mit einem aus RNA bestehenden Erbgut, verursacht. Wichtige Erreger aus dieser Gruppe von Viren sind u.a. das Humane Immundefizeinz-Virus (HIV), das Hepatitis-C-Virus (HCV), das Hepatitis-A-Virus, das Grippe-Virus (Influenza A, B und C) aber auch das Vogelpest-Virus, das vor kurzem neu identifizierte SARS-Virus, das Virus der Kinderlähmung (Poliovirus), das Masern-Virus, das Mumps-Virus, das Röteln-Virus und die Brechdurchfall-Viren (Rotavirus, Sapovirus und Norovirus).

Der Nachweis von RNA im Patientenmaterial findet bis dato in vier Schritten statt. Nach der Gewinnung der RNA aus dem Untersuchungsmaterial folgen die Schritte:
1. Umschreibung der RNA in copy-SNA (cDNA) durch RNA-abhängige DNA-Polymerasen, die sog. reversen Transkriptasen. Dafür bedarf es auch des Einsatzes eines Primers. Primer sind Oligonukleotide, die spezifisch für die zu amplifizierende Sequenz sind. Primer und die RNA-abhängige DNA-Polymerase ermöglichen die gezielte Amplifikation vom Erbgut, allerdings in DNA-Form.
2. Amplifikation der cDNA durch Polymerasekettenreaktion (PCR),
3. Aufreinigung des PCR-Produkts,
4. Sequenzierung des PCR-Produkts.

Für die Herstellung von RNA, beispielsweise zum Expressionsnachweis oder zur Herstellung von siRNA, erfolgt zusätzlich die Umschreibung des PCR-Produkts in RNA durch DNA-abhängige RNA-Polymerasen, wie z.B. die T7-Polymerase.
Die Durchführung dieser Schritte dauert in der Regel 18 bis 24 Stunden. Ebenfalls dazu benötigt wird auch ein gewisses "Know-How", was nicht allen Forschungseinrichtungen zugänglich ist.

Eine weitere Methode zur Amplifikation von RNA ist die Replikation durch RNA-abhängige RNA-Polymerasen wie z.B. die RNA-Polymerase des Bakteriophagen Qβ (auch Qβ - Replikase genannt), die RNA-Polymerase des Poliovirus oder des Hepatitis-C-Virus.

Das Genom des Bakteriophagen Qβ besteht aus einer einzelsträngigen (+)-RNA, die durch die RNA-abhängige RNA-Polymerase repliziert wird. Die Qβ - Replikase stellt eine (-)-Strang-RNA-Kopie des (+)-Stranges her, welche ebenso wie (+)-Stränge als Matrize fungieren kann. Auf diese Weise kann eine exponentielle Vervielfältigung erreicht werden. Das Qβ -System wird bereits zum Nachweis von Analyten, wie etwa Nukleinsäuren, verwendet (Chu et al. (1986), Nucleic Acids Research 14, 5591 - 5603; Lizardi et al (1988), Biotechnology 6, 1197 - 1202).

Diese RNA-abhängigen RNA-Polymerasen benötigen jedoch bestimmte RNA-Sequenzen zur Initiation der RNA-Replikation. Zudem sind sie für die Amplikation der RNA auch auf bestimmte Hilfsproteine angewiesen. Daher sind diese RNA-abhängigen RNA-Polymerasen nicht dazu in der Lage, heterologe RNA zu amplifizierten.

Die Markierung von RNA am 3'-Terminus ist nach dem Stand der Technik nur mit ATP durch die Verwendung von *E.coli* poly(A)- Transferase möglich. Verfahren zur Markierung von RNA mit CTP, UTP und GTP sind nicht bekannt.

Der Erfindung liegt daher die Aufgabe zugrunde, eine RNA-abhängige RNA-Polymerase sowie Verfahren und Kits zur Markierung und / oder zur Amplifikation von viraler, eukaryontischer und prokaryontischer einzel- und doppelsträngiger Ribonukleinsäure (RNA) bereitzustellen, die effizienter und schneller sind.

Erfindungsgemäß wird die Aufgabe gelöst durch eine RNA-abhängige RNA-Polymerase (im folgenden auch als RdRP oder 3D^{pol} bezeichnet) zur Amplifikation und / oder Markierung von RNA, wobei die RNA-abhängige RNA-Polymerase zu den Viren der Familie der *Caliciviridae* gehört und eine "Rechte-Hand-KonformaHon" aufweist und die Aminosäuresequenz der RNA-abhängigen RNA-Polymerase die folgenden Sequenzabschnitte umfasst:
a. XXDYS
b. GXPSG
c. YGDD
d. XXYGL
e. XXXXFLXRXX
mit der Bedeutung:
D: Aspartat
Y: Tyrosin
S: Serin
G: Glycin
P: Prolin
L: Leucin
F: Phenylalanin
R: Arginin
X: eine beliebige Aminosäure.

Unter einer sog. "Rechte-Hand-Konformation" ist dabei zu verstehen, dass die tertiäre Struktur (Konformation) des Proteins eine Faltung nach einer rechten Hand mit Finger (finger), Handfläche (palm) und Daumen (thumb), aufweist.

Bei dem Sequenzabschnitt "XXDYS" handelt es sich um das sog. A-Motiv. Das A-Motiv ist für die Diskriminierng zwischen Ribonukleosiden und Deoxyribonukleosiden zuständig.
Bei dem Sequenzabschnitt GXPSG" handelt es sich um das sog. B-Motiv. Das B-Motiv ist in allen Vertretern der Familie *Caliciviridae* konserviert.
Bei dem Sequenzabschnitt "YGDD" handelt es sich um das sog. C-Motiv. Das C-Motiv stellt die aktive Stelle des Enzyms dar. Dieses Motiv spielt bei der Koordinierung der Metallionen eine wichtige Rolle.
Bei dem Sequenzabschnitt "XXYGL" handelt es sich um das sog. D-Motiv. Das D- Motiv ist ein Merkmal der Template-abhängigen Polymerasen.

Bei dem Sequenzabschnitt "XXXXFLXRXX" handelt es sich um das sog. E-Motiv. Das E-Motiv ist ein Merkmal der RNA-abhängigen RNA Polymerasen, und kommt bei DNA-abhängigen RNA-Polymerasen nicht vor.

Die erfindungsgemäße RNA-abhängige RNA-Polymerase verfügt über die folgenden Funktionen:
- Mg²⁺- oder Mn²⁺-abhängige RNA-abhängige RNA-Polymeraseaktivität, keine DNA-Abhängigkeit
- Tomplate-Abhängigkelt
- primer-abhängige RNA-Synthese mit homopolymerer RNA (poly(A)-RNA, poly(C)-RNA, poly(G)-RNA bzw. poly(U)-RNA) als Matrize und bei Anwesenheit der entsprechenden Primer (d.h. oligo(U)-RNA, oligo(G)-RNA, oligo-(C)-RNA, bzw. oligo(A)-RNA)
- primer-unabhängige RNA-Synthese mit poly(C)-RNA als Matrize in Anwesenheit von erhöhten GTP-Konzentrationen
- Primer-abhängige oder Primer-unabhängige RNA-Synthese mit heteropolymerer RNA als Matrize
- Terminale Transferase-Aktivität mit einer Präferenz für UTP und CTP, was zum "Labelling" des 3'-Endes eines einzelsträngigen RNA-Templates führt.

Die Primer-abhängige Amplifikation der RNA erfolgt nur in der Anwesenheit eines Sequenzspezifischen Primers. Die Amplifikation ist Sequenz-abhängig und erfolgt durch Einbau von AMP, GMP, CMP oder UMP.
Die Primer-unabhängige Amplifikation der RNA erfolgt in der Abwesenheit eines Sequenzspezifischen Primers, Die Amplifikation ist Sequenz-abhängig und erfolgt durch Einbau von AMP, GMP, CMP oder UMP.

Durch die Terminale Transferase-Aktivität werden am 3'-Ende eines RNA-Stranges mehrere Nucleotide gleicher Art angehängt (z.B. ATP oder UTP oder CTP oder GTP), unabhängig von der Sequenz des RNA-Stranges.

Überraschenderweise ist die erfindungsgemäße RNA-abhängige RNA-Polymerase in der Lage, *in vitro* heterologe virale, eukaryontische und prokaryontische RNA als Template für die Amplifikationsreaktion zu nutzen. Sowohl positiv-strängige und negativ-strängige einzelsträngige als auch doppelsträngige RNA- ist als Template zur Amplifikation einsetzbar.

Vorzugsweise ist die erfindungsgemäße RNA-abhängige RNA-Polymerase eine RNA-abhängige RNA-Polymerase eines Virus aus der Familie der *Caliciviridae.* Das Genom der *Caliciviridae* besteht aus einem polyadenylierten (+)-strängigen RNA-Einzeistrang. *In vivo* schreibt die RNA-abhängige RNA-Polymerase des Calicivirus bei der viralen Replikation die genomische Calicivirus-RNA in eine (-) - strängige antisense-RNA (aRNA) um. Dabei entsteht ein RNA-aRNA-Hybrid. Die (-)-strängige aRNA dient im Anschluß als Templat zur Synthese neuer (+)-strängiger genomischer Virus-RNA.

In weiteren bevorzugten Ausführungsformen der Erfindung ist die RNA-abhängige RNA-Polymerase eine RNA-abhängige RNA-Polymerase eines humanen und / oder nicht-human pathogenen Calicivirus. Besonders bevorzugt handelt es sich um eine RNA-abhängige RNA-Polymerase eines Norovirus, Sapovirus, Vesivirus oder eines Lagovirus, z.B. die RNA-abhängige RNA-Polymerase des Norovirus-Stammes HuCV/NL/Dresden174/1997/GE oder des Sapovirus-Stammes pJG-Sap01 oder des Vesivirus-Stammes FCV/Dresden/2006/GE.

In besonders bevorzugten Ausführungsformen der Erfindung ist die RNA-abhängige RNA-Polymerase ein Protein mit einer Aminosäuresequenz gemäß **SEQ ID NO 1, SEQ ID NO 2** oder **SEQ ID NO 3,**
**SEQ ID NO 1** ist die Aminosäuresequenz einer Norovirus-RdRP.
**SEQ ID NO 2** ist die Aminosäuresequenz einer Sapovirus-RdRP.
**SEQ ID NO 3** ist die Aminosäuresequenz einer Vesivirus-RdRP.

SEQ ID NO 1:
MGGDSKGTYCGAPILGPGSAPKLSTKTKFWRSSTTPLPPGTYEPAYLGGK
DPRVKGGPSLQQVMRDQLKPFTEPRGKPPKPSVLEAAKKTIINVLEQTID
PPEKWSFTQACASLDKTTSSGHPHHMRKNDCWNGESFTGKLADQASKANL
MFEGGKNMTPVYTGALKDELVKTDKIYGKIKKRLLWGSDLATMIRCARAF
GGLMDELKAHCVTLPIRVGMNMNEDGPIIFERHSRYKYHYDADYSRWDST
QQRAVLAAALEIMVKFSSEPHLAQVVAEDLLSPSVVDVGDFKISINEGLP
SGVPCTSQWNSIAHWLLTLCALSEVTNLSPDIIQANSLFSFYGDDEIVST
DIKLDPEKLTAKLKEYGLKPTRPDKTEGPLVISEDLNGLTFLRRTVTRDP
AGWFGKLEQSSILRQMYWTRGPNHEDPSETMIPHSQRPIQLMSLLGEAAL
HGPAFYSKISKLVIAELKEGGMDFYVPRQEPMFRWMRFSDLSTWEGDRNL
APSFVNEDGVEVDKLAAALE

SEQ ID NO 2:
MKDEFQWKGLPVVKSGLDVGGMPTGTRYHRSPAWPEEQPGETHAPAPFGAGD
KRYTFSQTEMLVNGLKPYTEPTAGVPPQLLSRAVTHVRSYIETIIGTHRS
PVLTYHQACELLERTTSCGPFVQGLKGDYWDEEQQQYTGVLANHLEQAWD
KANKGIAPRNAYKLALKDELRPIEKNKAGKRRLLWGCDAATTLIATAAFK
AVATRLQVVTPMTPVAVGINMDSVQMQVMNDSLKGGVLYCLDYSKWDSTQ
NPAVTAASLAILERFAEPHPIVSCAIEALSSPAEGYVNDIKFVTRGGLPS
GMPFTSVVNSINHMIYVAAAILQAYESHNVPYTGNVFQVETVHTYGDDCM
YSVCPATASIFHAVLANLTSYGLKPTAADKSDAIKPTNTPVFLKRTFTQT
PHGVRALLDITSITRQFYWLKANRTSDPSSPPAFDRQARSAQLENALAYA
SQHGPVVFDTVRQIAIKTAQGEGLVLVNTNYDQALATYNAWFIGGTVPDP
VGHTEGTHKIVFEME

SEQ ID NO 3:
MKVTTQKYDVTKPDISYKGLICKQLDEIRVIPKGTRLHVSPAHTDDYDEC
SHQPASLGSGDPRCPKSLTAIVVDSLKPYCEKTDGPPHDILHRVQRMLID
HLSGFVPMNISSEPSMLAAFHKLNHDTSCGPYLGGRKKDHMIGGEPDKPL
LDLLSSKWKLATQGIGLPHEYTIGLKDELRPVEKVQEGKRRMIWGCDVGV
ATVCAAAFKGVSDAITANHQYGPVQVGINMDGPSVEALYQRIRSAAKVFA
VDYSKWDSTQSPRVSAASIDILRYFSDRSPIVDSAANTLKSPPIAIFNGV
AVKVTSGLPSGMPLTSVINSLNHCLYVGCAILQSLESRNIPVTWNLFSTF
DMMTYGDDGVYMFPMMFASVSDQIFANLTAYGLKPTRVDKSVGAIEPIDP
ESVVFLKRTITRTPHGIRGLLDRGSIIRQFYYIKGENSDDWKTPPKTIDP
TSRGQQLWNACLYASQHGPEFYNKVYRLAEKAVEYEELHFEPPSYHSALE
HYNNQFNGVDTRSDQIDASVMTDLHCDVFEVLE

Die RNA-abhängigen RNA-Polymerasen des Norovirus, des Sapovirus und des Vesivivirus sind rekombinant herstellbar durch Klonierung der die Norovirus-, Sapovirus bzw. Vesivirus-RdRP kodierenden Nukleinsäure in einen geeigneten Expressionsvektor, beispielsweise pET-28 (Novagen). Der Expressionsvektor mit der Gensequenz, die für die Virus-RdRP kodiert, wird zur Expression in einen geeigneten Wirtsorganismus eingebracht. Der Wirtsorganismus wird bevorzugt aus den üblicherweise für die Proteinexpression verwendeten prokaryontischen, bevorzugt *Eschericha coli,* oder eukaryontischen Wirtsorganismen, bevorzugt *Sacharomyces cerevisae* oder Insektenzellen (bevorzugt Sf9-Zellen), die mit rekombinanten Baculoviren infiziert worden sind, gewählt Dieser Wirtsorganismus enthält mindestens einen Expressionsvektor mit einer Gensequenz, die für die Virus-RdRP kodiert,

In bevorzugten Ausführungsformen wird die Norovirus-, Sapovirus- oder Vesivirus- Calicivirus-RdRP am N- oder C-Terminus mit einer Proteinsequenz fusioniert, welche die Aufreinigung des RdRP-Fusionsproteins nach der Expression in einem Wirtsorganismus erleichtert. Bevorzugt ist diese Sequenz ein sogenannter Histidin-Marker, bestehend aus einer Sequenz von mindestens 6 aufeinanderfolgenden Histidinen (His oder H). Ein solcher Histidin-Marker ermöglicht in bekannter Weise die Aufreinigung des Proteins affinitätschromatographisch auf einer Nickel- oder Kobalt-Säule.

Beispiele für mit einem Histidin-Marker fusionierte Ausführungsformen der RNA-abhängigen RNA-Polymerase sind die Proteine mit einer Aminosäuresequenz gemäß **SEQ ID NO 4, SEQ ID NO 5** oder **SEQ ID NO 6.**
**SEQ ID NO 4** ist die Aminosäuresequenz einer Norovirus-RdRP mit einem Histidin-Marker (His-tag).
**SEQ ID NO 5** ist die Aminosäuresequenz einer Sapovirus-RdRP mit einem Histidin-Marker (His-tag).
**SEQ ID NO 6** ist die Aminosäuresequenz einer Vesivirus-RdRP mit einem Histidin-Marker (His-tag).

SEQ ID NO 4:
MGGDSKGTYCGAPILGPGSAPKLSTKTKFWRSSTTPLPPGTYEPAYLGGK

DPRVKGGPSLQQVMRDQLKPFTEPRGKPPKPSVLEAAKKTIINVLEQTID
PPEKWSFTQACASLDKTTSSGHPHHMRKNDCWNGESFTGKLADQASKANL
MFEGGKNMTPVYTGALKDELVKTDKIYGKIKKRLLWGSDLATMIRCARAF
GGLMDELKAHCVTLPIRVGMNMNEDGPIIFERHSRYKYHYDADYSRWDST
QQRAVLAAALEIMVKFSSEPHLAQVVAEDLLSPSVVDVGDFKISINEGLP
SGVPCTSQWNSIAHWLLTLCALSEVTNLSPDIIQANSLFSFYGDDEIVST
DIKLDPEKLTAKLKEYGLKPTRPDKTEGPLVISEDLNGLTFLRRTVTRDP
AGWFGKLEQSSILRQMYWTRGPNHEDPSETMIPHSQRPIQLMSLLGEAAL
HGPAFYSKISKLVIAELKEGGMDFYVPRQEPMFRWMRFSDLSTWEGDRNL
APSFVNEDGVEVDKLAAALEHHHHHH

SEQ ID NO 5:
MKDEFQWKGLPVVKSGLDVGGMPTGTRYHRSPAWPEEQPGETHAPAPFGAGD
KRYTFSQTEMLVNGLKPYTEPTAGVPPQLLSRAVTHVRSYIETTIGTHRS
PVLTYHQACELLERTTSCGPFVQGLKGDYWDEEQQQYTGVLANHLEQAWD
KANKGIAPRNAYKLALKDELRPIEKNKAGKRRLLWGCDAATTLIATAAFK
AVATRLQVVTPMTPVAVGINMDSVQMQVMNDSLKGGVLYCLDYSKWDSTQ
NPAVTAASLAILERFAEPHPIVSCAIEALSSPAEGYVNDIKFVTRGGLPS
GMPFTSVVNSINHMIYVAAAILQAYESHNVPYTGNVFQVETVHTYGDDCM
YSVCPATASIFHAVLANLTSYGLKPTAADKSDAIKPTNTPVFLKRTFTQT
PHGVRALLDITSITRQFYWLKANRTSDPSSPPAFDRQARSAQLENALAYA
SQHGPVVFDTVRQIAIKTAQGEGLVLVNTNYDQALATYNAWFIGGTVPDP
VGHTEGTHKIVFEMEHHHHHH

SEQ ID NO 6:
MKVTTQKYDVTKPDISYKGLICKQLDEIRVIPKGTRLHVSPAHTDDYDEC
SHQPASLGSGDPRCPKSLTAIVVDSLKPYCEKTDGPPHDILHRVQRMLID
HLSGFVPMNISSEPSMLAAFHKLNHDTSCGPYLGGRKKDHMIGGEPDKPL
LDLLSSKWKLATQGIGLPHEYTIGLKDELRPVEKVQEGKRRMIWGCDVGV
ATVCAAAFKGVSDAITANHQYGPVQVGINMDGPSVEALYQRIRSAAKVFA
VDYSKWDSTQSPRVSAASIDILRYFSDRSPIVDSAANTLKSPPIAIFNGV
AVKVTSGLPSGMPLTSVINSLNHCLYVGCAILQSLESRNIPVTWNLFSTF
DMMTYGDDGVYMFPMMFASVSDQIFANLTAYGLKPTRVDKSVGAIEPIDP
ESVVFLKRTITRTPHGIRGLLDRGSIIRQFYYIKGENSDDWKTPPKTIDP
TSRGQQLWNACLYASQHGPEFYNKVYRLAEKAVEYEELHFEPPSYHSALE
HYNNQFNGVDTRSDQIDASVMTDLHCDVFEVLEHHHHHH

Bestandteil der Erfindung ist auch die Verwendung einer erfindungsgemäßen RNA-abhängigen RNA-Polymerase zur Amplifikation und / oder Markierung von RNA.

Bestandteil der Erfindung ist auch ein Verfahren zur Amplifikation von RNA mittels einer erfindungsgemäßen RNA-abhängigen RNA-Polymerase mit den Schritten
a. Anlagerung der RNA-abhängigen RNA-Polymerese an die RNA-Matrize in der Anwesenheit oder Anwesenheit eines Oligonukleotid-Primers, z.B. eines Oligo-RNA-Primers oder Oligo-DNA-Primers,
b. Umschreiben der RNA-Matrize in antisense-RNA durch die RNA-abhängige RNA-Polymerase,
c. Trennung des RNA/antisense-RNA-Duplexes in einzelne RNA-Stränge
sowie ein Kit zur Durchführung des Verfahrens zur Amplifikation von RNA, mindestens bestehend aus
a. einer erfindungsgemäßen RNA-abhängigen RNA-Polymerase
b. einem geeigneten Reaktions-Puffer
c. NTPs
d. ggf. RNase-Inhibitor
e. ggf. Stoplösung

Die RNA-Matrize wird beim erfindungsgemäßen Verfahren vorzugsweise in Mengen von 1 µg bis 4 µg pro 50 µl-Reaktionsansatz eingesetzt. Die Konzentration der eingesetzten Ribonukleotide ATP, CTP, GTP und UTP (NTPs) beträgt vorzugsweise zwischen 0,1 µmol/l und 1 µmol/l, besonders bevorzugt 0,4 µmol/l. Auch NTP-Analoga sind beim erfindungsgemäßen Verfahren zur RNA-Amplifikation einsetzbar, beispielsweise fluoreszenzmarkierte NTPs, Biotin-markierte NTPs oder radioaktiv-markierte NTPs Wie [P³²]-markierte NTPs. Die Konzentration der erfindungsgemäßen RdRP beträgt bevorzugt zwischen 1 µmol/l und 3 µmol/l.

In einer besonders bevorzugten Ausführungsform enthält das Kit
a. 150 µmol/l rekombinant hergestellter Norovirus-RdRP gemäß **SEQ ID NO 1** oder **SEQ ID NO 4** und/oder Sapovirus-RdRP gemäß **SEQ ID NO 2** oder **SEQ ID NO 5** und/oder Vesivirus-RdRP gemäß **SEQ ID NO 3** oder **SEQ ID NO 6**
b. als Puffer: 50 mmol/l HEPES, pH 8.0, 3 mmol/l Magnesiumacetat oder Manganchlorid (MnCl₂), 4 mM DTT
c. 10 mmol/l ATP, 10 mmol/l CTP, 10 mmol/l GTP, 10 mmol/l UTP
d. RNese-Inhibitor
e. als Stoplösung: 4 mol/l Ammoniumacetat, 100 mmol/l EDTA

Das Verfahren wird vorzugsweise bei einem pH-Wert zwischen 6,5 und 8,5 und bei einer Temperatur zwischen 20°C und 40°C durchgeführt. Eine bevorzugte Ausführungsform des Verfahrens zur RNA-Amplifikation wird bei 30°C mit Norovirus-RdRP oder bei 37°C mit Sapovirus-RdRP unter folgenden Pufferbedingungen durchgeführt: 50 mmol/l HEPES, pH 8.0, 3 mmol/l Magnesiumacetat oder Manganchlorid, 4 mmol/l DTT.

In einer Ausführungsform der Erfindung erfolgt die Trennung des RNA / antisense-RNA-Duplexes in einzelne RNA-Stränge durch Hitzedenaturierung, durch chemische Denaturierung und / oder enzymatisch, z.B. mit einem Enzym mit der Fähigkeit, doppelsträngige RNA in einzelsträngige RNA aufzutrennen wie z.B. eine Helikase.

Durch die Strangtrennung liegt wieder einzelsträngiges Template vor und eine weitere Amplifikationsrunde kann eingeleitet werden.
Durch den Einsatz eines Enzyms mit der Fähigkeit, doppelsträngige RNA in einzelsträngige RNA aufzutrennen, kann die Reaktion isotherm gehalten werden.

Bestandteil der Erfindung ist ein entsprechendes Kit zur Amplifikation von RNA, das zusätzlich zum erfindungsgemäßen Kit ein Enzym mit der Fähigkeit, doppelsträngige RNA in einzelsträngige RNA, aufzutrennen wie z.B. eine Helikase, enthält.

Eine Ausführungsform der Erfindung ist ein Verfahren zur primerabhängigen Amplifikation von RNA, wobei mindestens ein Primer, der mit einem Abschnitt der RNA-Matrize hybridisiert, eingesetzt wird und nach der Anlagerung der erfindungsgemäßen RNA-abhängigen RNA-Polymerase der Primer durch die RNA-abhängige RNA-Polymerase entsprechend der Sequenz der RNA-Matrize verlängert wird.

Als Primer wird bevorzugt ein RNA-Primer oder ein DNA-Primer, z.B. mit einer Länge von 20 bis 25 Basen, vorzugsweise in einer Konzentration von 0,1 bis 1 µmol/l eingesetzt. Als RNA-Matrize ist dabei z.B. virale, prokaryontische und eukaryontische RNA einsetzbar.

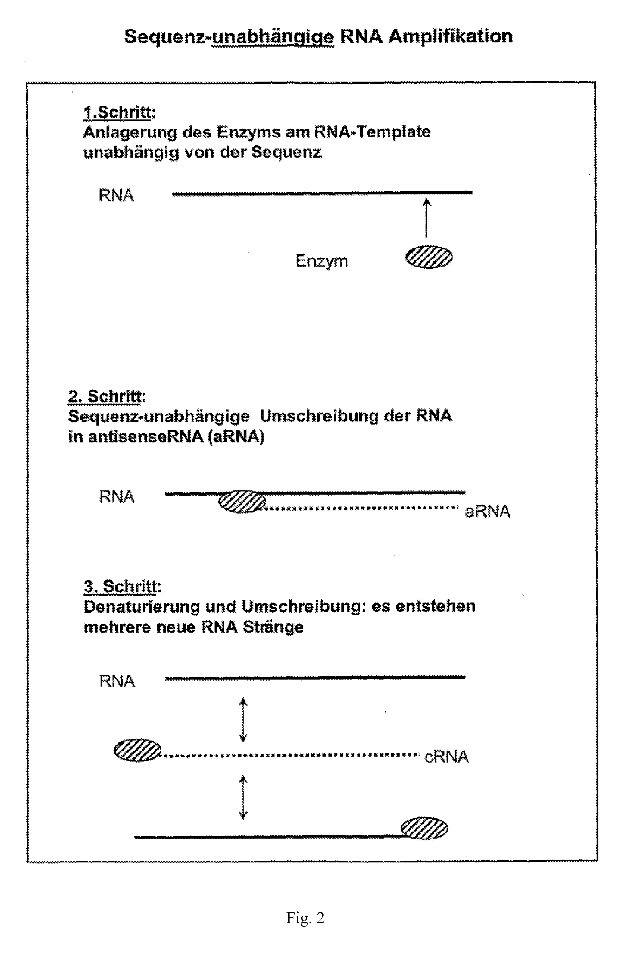

Der Ablauf der primerabhängigen RNA-Amplifikation einer einzelsträngigen RNA-Matrize unter Einsatz eines RNA-Primers ist in **Figur 2** schematisch dargestellt.

Vorzugsweise wird bei polyadenylylierter RNA, polyurydylitierter RNA, polyguanylylierter RNA oder polycytidylylierter RNA als Primer, ein poly-U-RNA, poly-A-RNA, poly-C-RNA oder poly-G-RNA-Primer eingesetzt. Mit Hilfe des poly-U-RNA-Primers ist eine spezifische Amplifikation der gesamten zellulären mRNA möglich. Vorzugsweise wird dafür ein Poly-U-Primer von 20 bis 24 Basen Länge eingesetzt. Die amplifizierte zelluläre mRNA kann Anschluss beilspielsweise mit Hilfe der sog. Microarray-Verfahren untersucht werden.

Das erfindungsgemäße Verfahren zur sequenzspezifischen RNA-Amplifikation wird vorteilhaft auch zum Nachweis viraler RNA in Patientenmaterial verwendet.

Hierfür wird die gesamte zelluläre RNA aus dem Patientenmaterial gewonnen und unter Einsatz eines RNA-Primers, der spezifisch mit einem bestimmten Abschnitt viraler RNA hybridisiert, wird im Patientenmaterial vorhandene virale RNA amplifiziert.

Als Patientenmaterial wird beispielsweise Liquor, Blut, Plasma oder Körperflüssigkeiten eingesetzt.

Auch geeignet ist das Verfahren zum Nachweis von RNA-Viren mit einem poly-A-Schwanz am 3'-Ende des Genoms, DNA-Viren und viralen mRNA-Transkripten.

Bestandteil der Erfindung ist ebenfalls ein Kit zur Durchführung des Verfahrens zur primerabhängigen Amplifikation von RNA, mindestens bestehend aus
a. einer erfindungsgemäßen RNA-abhängigen RNA-Polymerase
b. einem geeigneten Reaktions-Puffer
c. NTPs
d. ggf. RNase-Inhibitor
e. ggf. Stoplösung
f. Primer
In einer besonders bevorzugten Ausführungsform enthält das Kit
a. 150 rekombinant hergestellter µmol/l Norovirus-RdRP gemäß **SEQ ID NO 1** oder **SEQ ID NO 4** und/oder Sapovirus-RdRP gemäß **SEQ ID NO 2** oder **SEQ ID NO 5** und/oder Vesivirus-RdRP gemäß **SEQ ID NO 3** oder **SEQ ID NO 6**
b. als Puffer: 50 mmol/l HEPES, pH 8.0, 3 mmol/l Magnesiumacetat oder Manganchlorid (MnCl₂), 4 mM DTT
c. 10 mmol/l ATP, 10 mmol/l CTP, 10 mmol/l GTP, 10 mmol/l UTP
d. RNase-Inhibitor
e. als Stoplösung: 4 mol/l Ammoniumacetat, 100 mmol/l EDTA
f. 0,1 bis 3 µM Primer (homopolymeres oder heteropolymeres RNA- oder DNA-Oligonukleotid mit einer Länge von 15 bis 20 Basen)

Ebenfalls Teil der Erfindung ist ein Verfahren zur primerunabhängigen) Amplifikation von RNA, wobei kein Primer, der mit einem Abschnitt der RNA-Matrize hybridisiert, eingesetzt und durch die RNA-abhängige RNA-Polymerase entsprechend der Sequenz der RNA-Matrize verlängert wird, sowie ein Kit zur primerunabhängigen Amplifikation von RNA, mindestens bestehend aus
a. einer erfindungsgemäßen RNA-abhängigen RNA-Polymerase
b. einem geeigneten Reaktions-Puffer
c. NTPs
d. ggf. RNase-Inhibitor
e. ggf. Stoplösung.

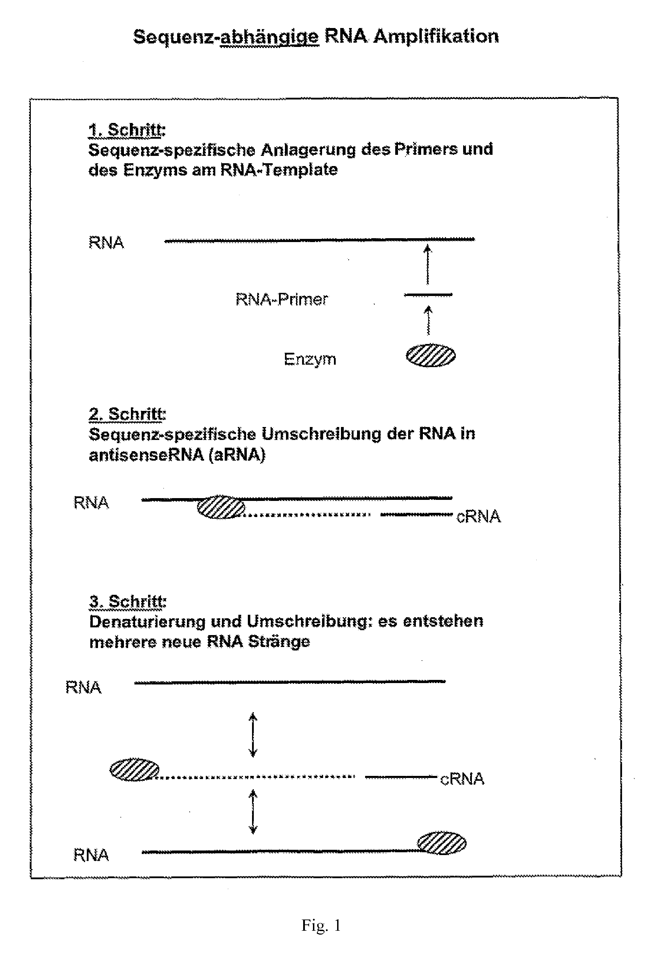

In dieser Ausgestaltung des erfindungsgemäßen Verfahrens erfolgt die Anlagerung der RNA-abhängigen RNA-Polymerase an die RNA-Matrize primerunabhängig. Der Ablauf der sequenzunabhängigen RNA-Amplifikation einer einzelsträngigen RNA-Matrize ohne Einsatz eines RNA-Primers ist in **Figur 1** schematisch dargestellt.

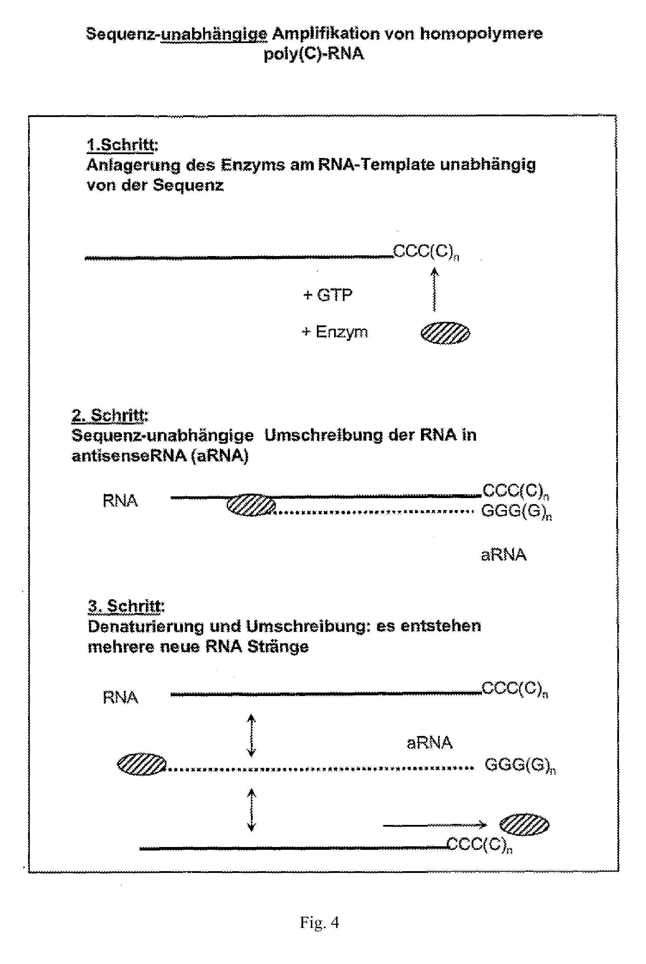

Eine weitere Ausführungsform der Erfindung ist ein Verfahren zur primerunabhängigen Amplifikation von poly(C)-RNA, dadurch gekennzeichnet, dass kein Primer, der mit einem Abschnitt der RNA-Matrize hybridisiert, eingesetzt wird, dass GTP, vorzugsweise 50 µM, als einziger Nukleotid verwendet wird und durch die RNA-abhängige RNA-Polymerase entsprechend der Sequenz der RNA-Matrize verlängert wird.
Der Schematische Ablauf der sequenz-unabhängigen RNA-Synthese ausgehend von einer poly(C)-RNA in Anwesenheit von 50 µM GTP ist in **Figur 4** dargestellt.

Die Anwendungsmöglichkeiten der erfindungsgemäßen Verfahren zur Amplifkation von RNA sind vielfältig, Zum einem ist der direkte Nachweis viralen Erbgutes In Patientenmaterial möglich. Hiefür wird die gesamte zelluläre RNA gewonnen und unter Einsatz eines RNA-Primers eine beliebige RNA-Sequenz spezifisch amplifiziert. Der Nachweis der viralen Nukleinsäure erfolgt dann durch Hybridisierung an spezifischen Sonden. Eine andere Anwendung betrifft die unspezifische (RNA-Primer-unabhängige) Amplifikation der RNA, die ermöglichen kann, bisher nicht identifizierte Viren bzw. neue virale Varianten zu identifizieren.

Eine weitere Anwendung betrifft die Microarray-Technologie. Diese hat den differenziellen Nachweis der zellulären Expression zum Ziel. Dies geschieht durch den Nachweis der sog. zellulärer Transkripte, d.h. der mRNA. Die Erfindung ermöglicht ausgehend aus einem zellulären Gemisch, die mRNA spezifisch durch den Einsatz eines poly(U)-Oligonukleotid zu amplifizieren, um anschließend auf Microarrays durch Hybridisierung den Nachweis zu erbringen.

Ein anderes Anwendungsgebiet der Erfindung ist die Herstellung von siRNA in vitro. Dies erfolgt bis dato mit Hilfe der T7-Polymerase. Es wird dafür
1. zwei komplementäre DNA-doppelstränge mittels PCR unter dem Einsatz eines T7-Promotor-Primer synthetisiert,
2. nach Umschreibung der DNA mit Hilfe der T7-Polymerase, werden beide resultierende komplementäre RNA-Stränge hybridisiert,
3. dann werden mittels Nase I beide Stränge verdaut.

Diese Schritte dauern nach dem Stand der Technik in der Regel 24 bis 48 Stunden, und benötigen ebenso das notwendige "Know-How". Die Erfindung ermöglicht demgegenüber in 1,5 Stunden und ausgehend von einer RNA-Sequenz die direkte, effiziente und einfache Herstellung doppelsträngiger RNA, ohne die Notwendigkeit eines PCR-Schrittes, der *in vitro* Transkription und der Hybridisierung der RNA (die eventuell sub-optimal verlaufen kann).

Bestandteil der Erfindung ist auch ein Verfahren zur Markierung von RNA mittels einer erfindungsgemäßen RNA-abhängigen RNA-Polymerase mit den Schritten
a. Anlagerung der RNA-abhängigen RNA-Polymerase an die zu markierende RNA,
b. Anhängen mindestens eines Nukleotids an das 3'-Ende der zu markierenden RNA.
sowie ein Kit zur Durchführung des erfindungsgemäßen Verfahrens zur Markierung von RNA, mindestens bestehend aus
a. einer erfindungsgemäßen RNA-abhängigen RNA-Polymerase
b. einem geeigneten Reaktions-Puffer
c. CTP oder UTP oder ATP oder GTP
d. ggf. RNase-Inhibitor
e. ggf. Stoplösung.

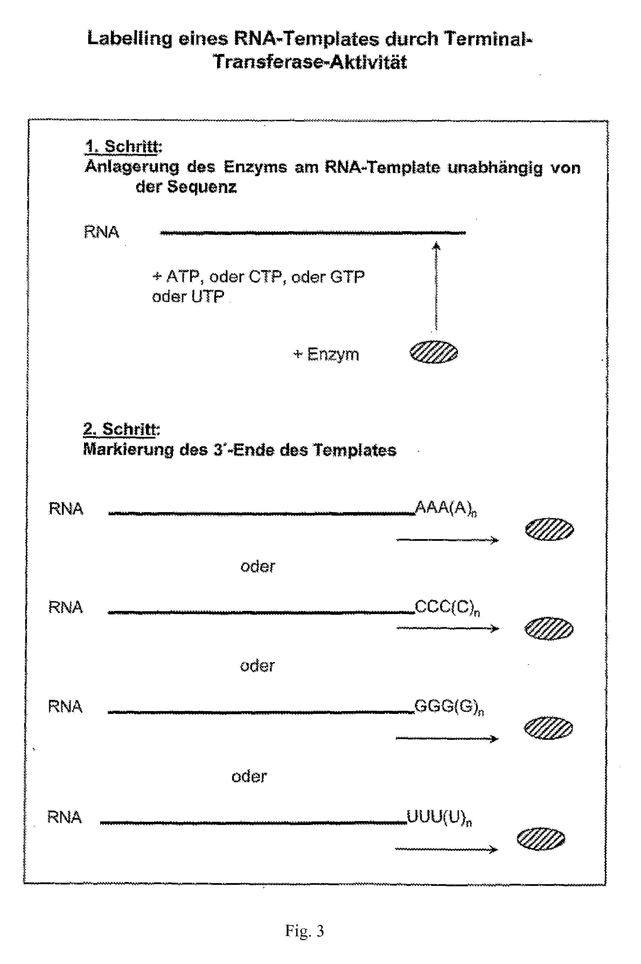

Der Ablauf des Labelling des 3'-Endes des RNA-Templates ist schematisch in Figur 3 dargestellt.

In einer besonders bevorzugten Ausführungsform enthält das Kit
g. 150 rekombinant hergestellter µmol/l Norovirus-RdRP gemäß **SEQ ID NO 1** oder **SEQ ID NO 4** und/oder Sapovirus-RdRP gemäß **SEQ ID NO 2** oder **SEQ ID NO 5** und/oder Vesivirus-RdRP gemäß **SEQ ID NO 3** oder **SEQ ID NO 6**
h. als Puffer: 50 mmol/l HEPES, pH 8.0, 3 mmol/l Magnesiumacetat oder Manganchlorid (MnCl₂), 4 mM DTT
i. 10 mmol/l ATP, und 10 mmol/l CTP, und 10 mmol/l GTP, und 10 mmol/l UTP,
j. RNase-Inhibitor
k. als Stoplösung.: 4 mol/l Ammoniumacetat, 100 mmol/l EDTA

Anhand der folgenden Figuren und Ausführungsbeispiele wird die Erfindung zäher erläutert. Dabei zeigen
- **Figur 1:**: **Schematische Ablauf der sequenz-abhängigen RNA-Amplifikation**
- **Figur 2:**: **Schematischer Ablauf der sequenz-unabhängigen RNA-Ampliflkation**
- **Figur 3:**: **Schematischer Ablauf des Labelling des 3'-Endes des RNA-Templates**
- **Figur 4:**: **Schematischer Ablauf der sequenz-unabhängigen RNA- Synthese ausgehend von einer poly(C)-RNA In Anwesenheit von 50 µM GTP**

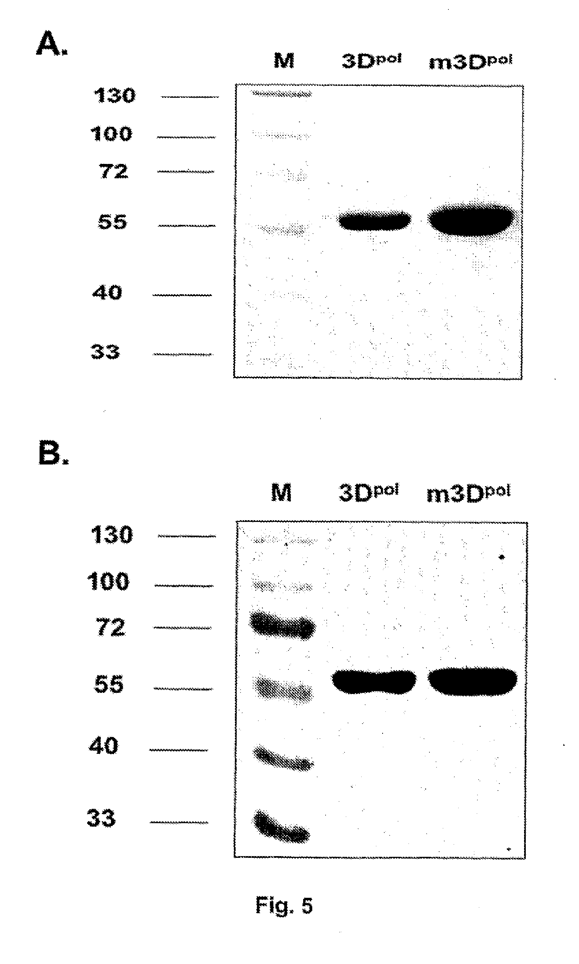

- **Figur 5:**: **Expression und Reinigung von Norovirus 3D^{pol} in *E*. *coli*.**
**(A)** SDS-PAGE-Anadyse von in *E*. *coli* exprimierter, gereinigter, rekombinanter Norovirus 3D^{pol} mit einem C-terminalen His-tag. 3D^{pol}, Wildtyp- Norovirus 3D^{pol}. m3D^{pol}, Norovirus 3D^{pol} mit einer Mutation in der 'active site' (YGD_{343G}D_{344G}). M, Molekulargewichtsmarker (KDa).
**(B)** Western-Blot-Analyse von gereinigter rekombinanter Norovirus 3D^{pol} unter Verwendung eines Peffia-Histidin-Antikörpers (monoklonaler Maus-Antikörper, Qiagen) zur Detektion von Proteinen mit einem C-terminalen His-tag. 3D^{pol}, Wildtyp-Norovirus 3D^{pol}. Norovirus 3D^{pol} mit einer Mutation in der 'active site' (YGD_{343G}D_{344G})- M, Molekulargewichtsmarker (KDa).

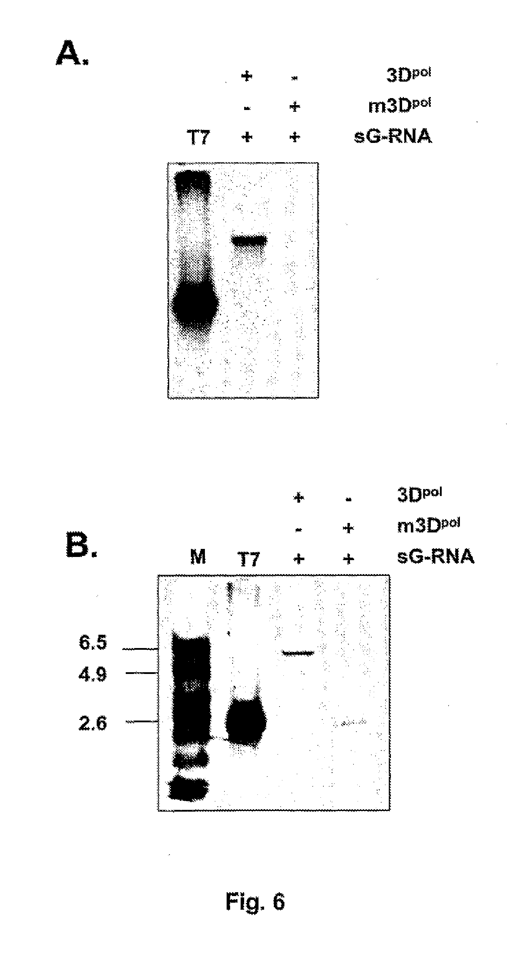

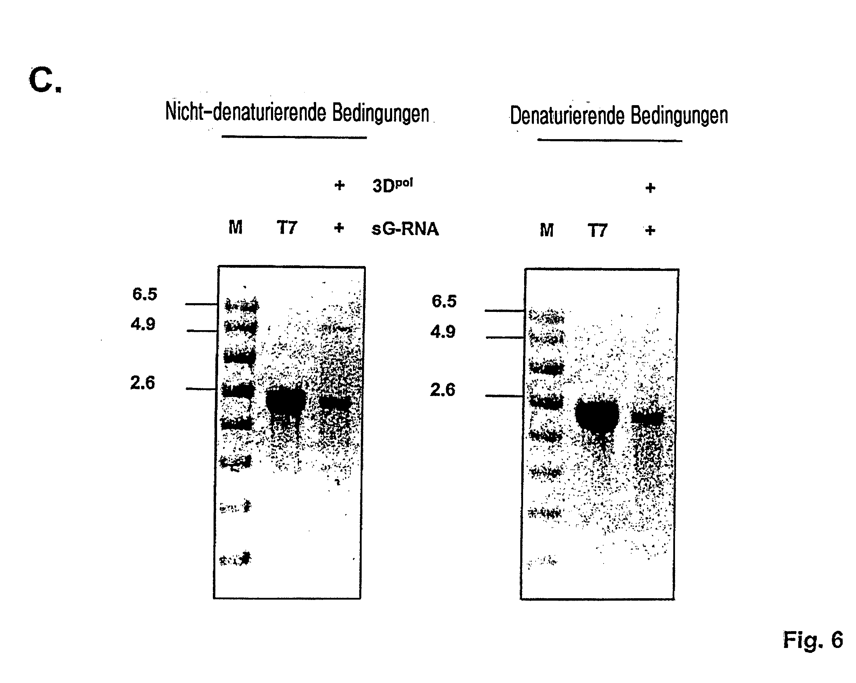

- **Figur 6:**: **RNA Synthese mit Norovirus 3D**^{pol}.
**(A)** Die RNA-Synthese wurde in Anwesenheit einer synthetischen subgenomischen RNA (sG-RNA) als Template sowie in Anwesenheit von Wildtyp-Norovirus 3D^{pol} oder einer Norovirus 3D^{pol} mit einer Mutation in der 'active site' (YGD_{343G}D_{344G}) wie angegeben untersucht. Die Reaktionsprodukte wurden auf einem nicht-denaturierenden Agarosegel analysiert und mittels Autoradiographie visualisiert. T7, synthetische subgenomische RNA, die durch In vitro Transkription mit T7 hergestellt wurde.
**(B)** Ethidiumbromid-Färbung desselben Gels und Visualisierung der Reaktionsprodukte durch UV-Transillumination. Die bei der Reaktion mit m3D^{pol} anstelle von Wildtyp 3D^{pol} sichtbare verbleibende Bande stammt von dem in der Reaktion eingesetzten synthetischen subgenomischen RNA-Template, M, RNA Molekulargewichtsmarker.
**(C)** Strangseparationsanalyse des Reaktionsprodukts der *in vitro* RNA-Synthese mit Norovirus 3D^{pol} Das Reaktionsprodukt wurde durch RNA-Synthese mit Norovirus 3D^{pol} (3D^{pol}) mit einer synthetischen subgenomischen RNA (sG-RNA) als Template hergestellt. Die Reaktionsprodukte wurden auf nicht-denaturierenden (0.5 M; links) und denaturierenden (1.25 M; rechts) Formaldehyde-Agarosegelen sichtbar gemacht. T7, synthetische subgenomische RNA, die mittels *in vitro* Transkription mit T7 hergestellt wurde. M, RNA Molekutargewichtsmarker.

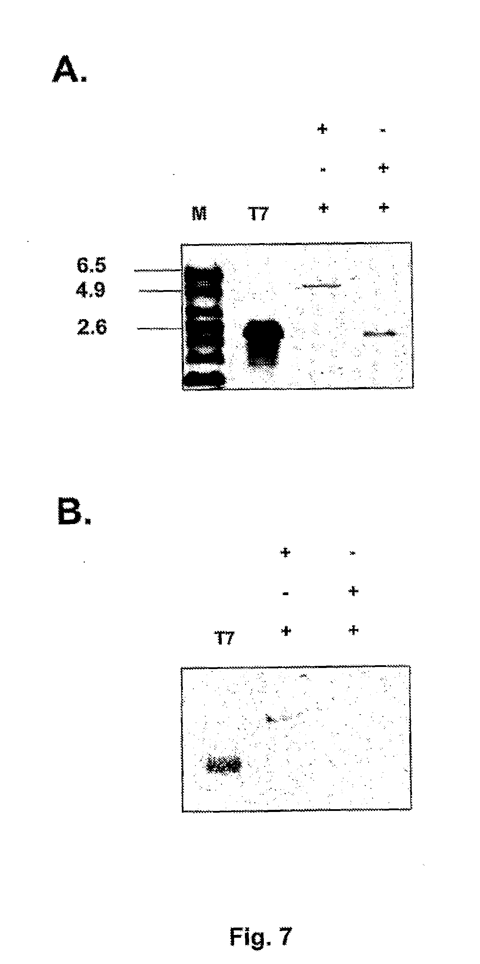

- **Figur 7:**: **Northern-Blot Analyse der Norovirus 3D^{pol} Syntheseprodukte.**
**(A)** Die RNA-Synthese wurde in Anwesenheit einer synthetischen subgenomischen RNA (sG-RNA) als Template sowie in Anwesenheit von Wildtyp-Norovirus 3D^{pol} oder einer Norovirus 3D^{pol} mit einer Mutation in der 'active site' (YGD_{343G}D_{344G}) wie angegeben durchgeführt. Die Reaktionsprodukte wurden auf einem nicht-denaturierenden Agarosegel analysiert und nach Ethidiumbromidfärbung mittels UV-Transillumination visualiert. T7, synthetische subgenomische RNA, die mittels *in vitro* Transkription mit T7 hergestellt wurde. M, RNA Molekulargewichtsmarker. Die bei der Reaktion mit m3D^{pol} anstelle von Wildtyp 3D^{pol} sichtbare verbleibende Bande stammt von dem in der Reaktion eingesetzten Template.
**(B)** Hybridisierung einer (-)-strängigen RNA-Probe mit den Syntheseprodukten der Norovirus 3D^{pol}. T7, synthetische subgenomische RNA, die mittels *in vitro* Transkription mit T7 hergestellt wurde. sG-RNA, als Template eingesetzte synthetische subgenomische RNA. 3D^{pol}, Wildtyp-Norovirus 3D^{pol}. Norovirus 3D^{pol} mit einer Mutation in der 'active site' (YGD_{343G}D_{344G}).

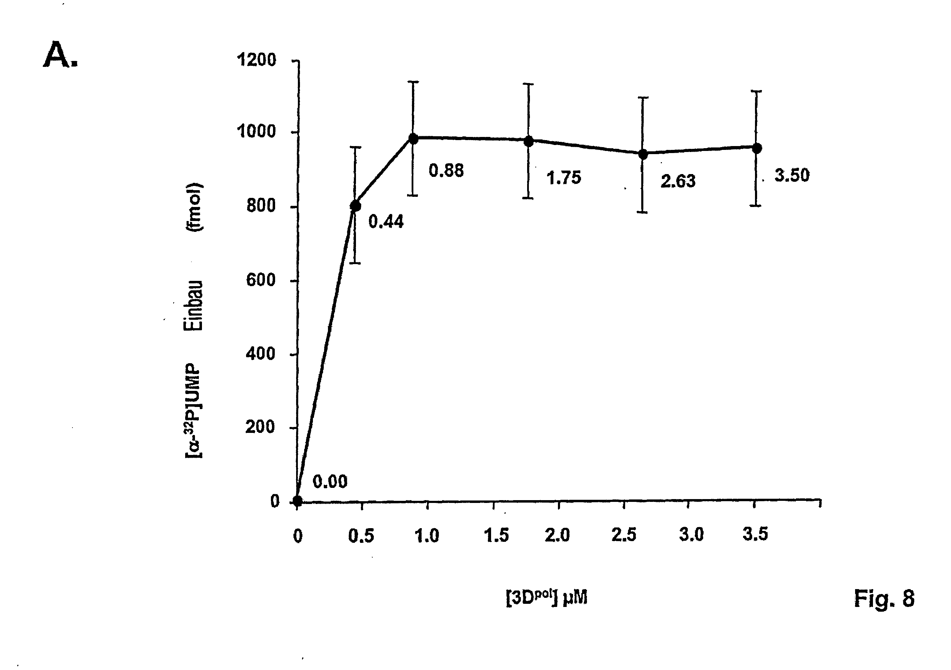

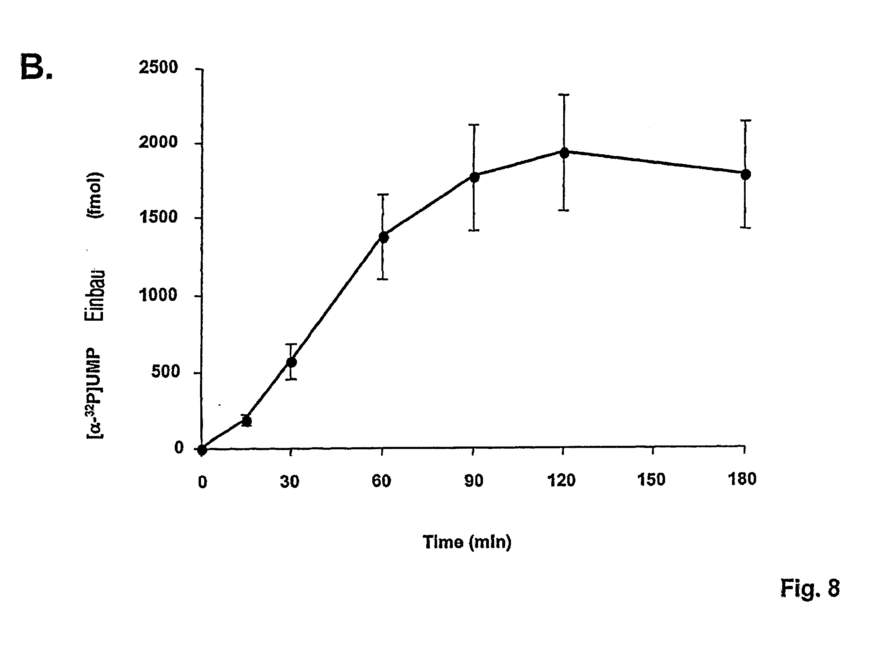

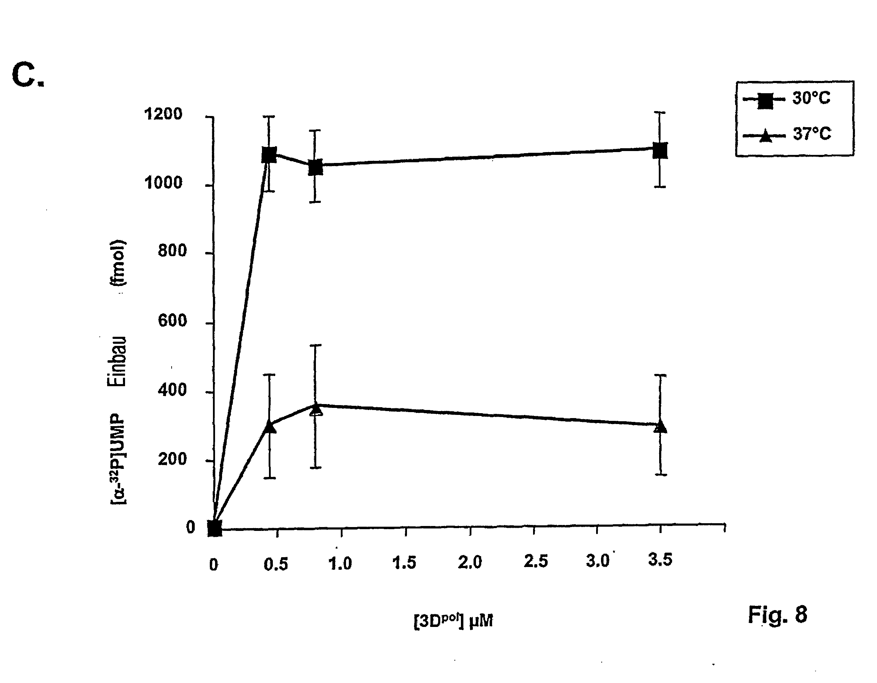

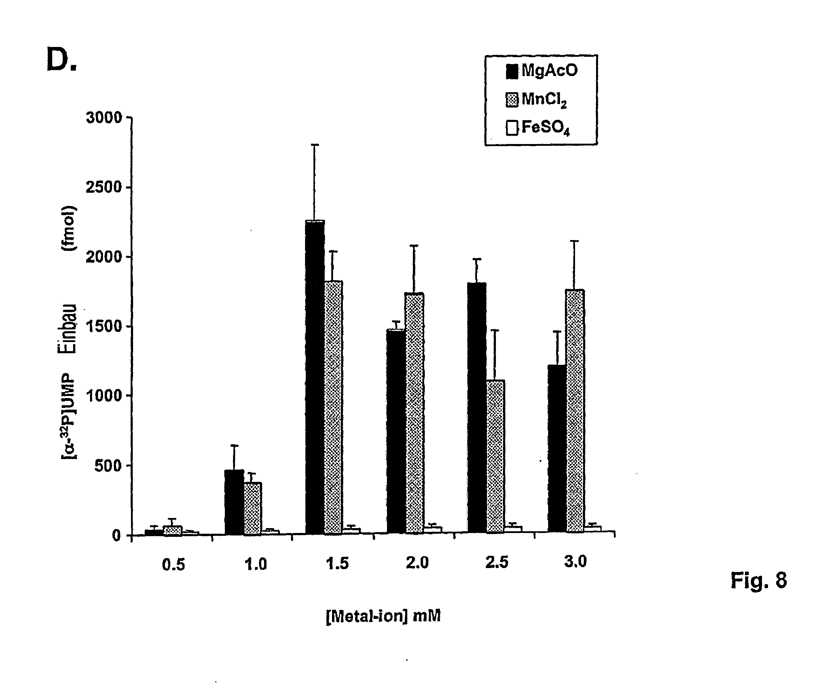

- **Figur 8:**: **Analyse der Konzentrationsabhängigkeit, der Temperaturabhängigkeit und des Zeitverlaufs der Norovirus 3D^{pol}-Aktivität.** Bei allen Reaktionen wurde subgenomische RNA als Template eingesetzt
**(A)** Konzentrationsabhängige Aktivität der Norovirus 3D^{pol}. Norovirus 3D^{pol} Konzentration (µM) wie angegeben. Die Reaktion wurde für 2 h bei 30°C durchgeführt. Für jede Konzentration sind Mittelwert und Standardabweichung dreier unabhängiger Experimente gezeigt.
**(B)** Zeitverlaufsanalyse der Norovirus 3D^{pol}-Aktivität. Einbau von [α-³²P]UMP wie angegeben. Die Reaktion wurde bei 30°C durchgeführt und 3 µM Norovirus 3D^{pol} wurden eingesetzt. Für jeden Zeitpunkt sind Mittelwert und Standardabweichung dreier unabhängiger Experimente Experimenten gezeigt.
**(C)** Temperaturabhängige Aktivität der Norovirus 3D^{pol}. Einbau von [α-³²P]UMP wie angegeben. Die Reaktionszeit war 2 h. Für jede Konzentration von Norovirus 3D^{pol} sind Mittelwert und Standardabweichung dreier unabhängiger Experimente gezeigt.
**(D)** Metallionen-Abhängigkeit der Norovirus 3D^{pol}-Aktivität. Die RNA-Synthese wurde über 2 h bei 30°C in Gegenwart von 3 µM Norovirus 3D^{pol}, subgenomischer RNA als Template (0.024 µM) und steigender Konzentrationen (0.5 bis 3 mM) von MgAcO, MnCl₂ oder FeSO₄ durchgeführt Einbau von [α-³²P]UMP wie angegeben. Für jede Konzentration sind Mittelwert und Standardabweichung dreier unabhängiger Experimente gezeigt.

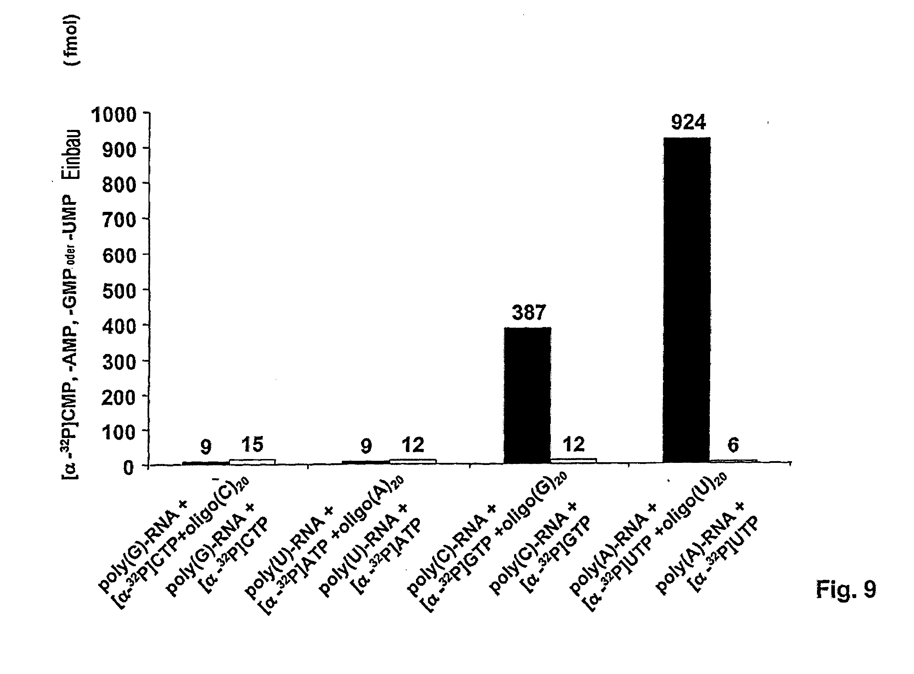

- **Figur 9:**: **Primer-abhänglge initiation der RNA Synthese auf hompolymeren Templaten.** Die RNA-Synthese wurde in Anwesenheit (schwarze Balken) oder in Abwesenheit (graue Balken) eines komplementären RNA-Oligonukleotid-Primers durchgeführt. Der Einbau von [α-³²P]CMP, [α-³²P]AMP, [α-³²P]GMP, oder [α-³²P]UMP wurde nach TCA-Präzipitation und Sammeln von G/C-Glassfasefiltern gemessen. Die Einbauwerte sind angegeben,

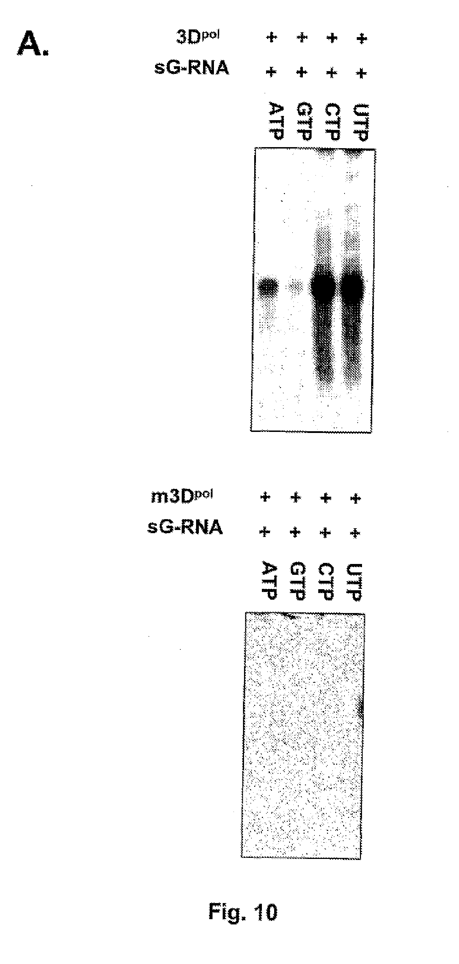

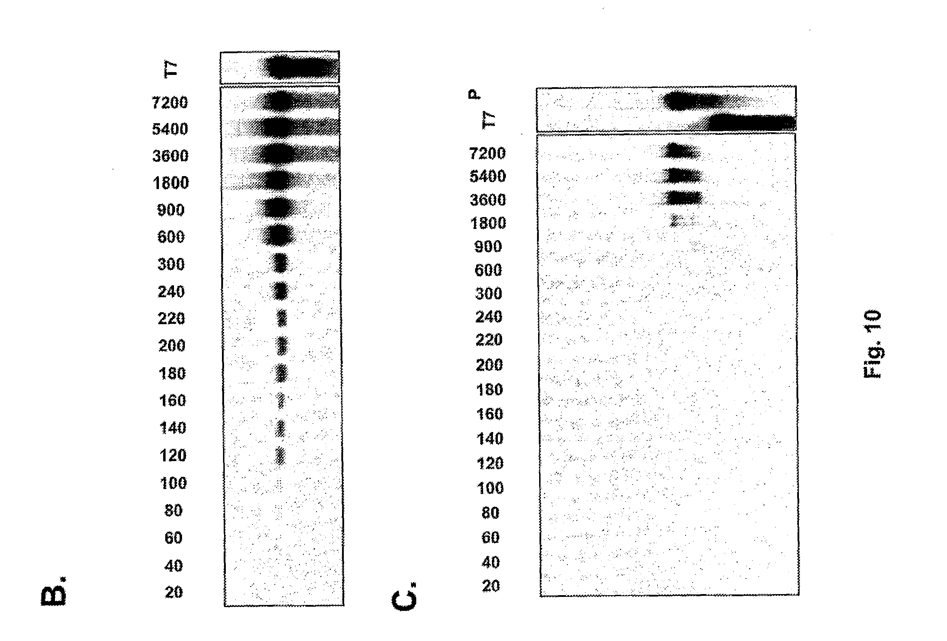

- **Figur 10:**: **Terminale Transferase - Aktivität der Norovirus 3D**^{pol}
**(A)** Zur Untersuchung der Terminalen Transferase - Aktivität wurde 3 µM Norovirus 3D^{pol} über 2 h bei 30°C in Gegenwart von subgenomischer RNA (sG-RNA) als Template (0.024 µM) und [α-³²P]ATP, [α-³²P]GTP, [α-³²P]CTP oder [α-³²P]UTP wie angegeben inkubiert. Die Reaktionsprodukte wurden auf nicht-denaturierenden Agarosegelen analysiert und mittels Autoradiographie visualisiert,
**(B)** Zeitverlaufsexperiment der Norovirus 3D^{pol} Terminalen Transferase - Aktivität an subgenomischer Norovirus-RNA als Template, Die Reaktion wurde wie unter (A) beschrieben, jedoch unter Verwendung von [α-³²P]UTP als gelabeltern Nukleotid durchgeführt, zu den angegebenen Zeiten (sec) gestoppt, und die Reaktionsprodukte wurden auf nicht-denaturierenden Agarosegelen mittels Autoradiographie visualisiert. T7, synthetische subgenomische RNA, die mittels *in vitro* Transkription mit T7 hergestellt wurde.
(C) Zeitverlaufsexperiment der RNA-Synthese mit 3 µM Norovirus 3D^{pol} unter Verwendung von subgenomischer RNA als Template. Die Reaktion wurde bei 30°C inkubiert, zu den angegebenen Zeiten (sec) gestoppt und die Reaktionsprodukte wurden auf nicht-denaturierenden Agarosegelen mittels Autoradiographie visualisiert T7, synthetische subgenomische RNA, die mittels in *vitro* Transkription mit T7 hergestellt wurde. P, Norovirus 3D^{pol} Syntheseprodukt.

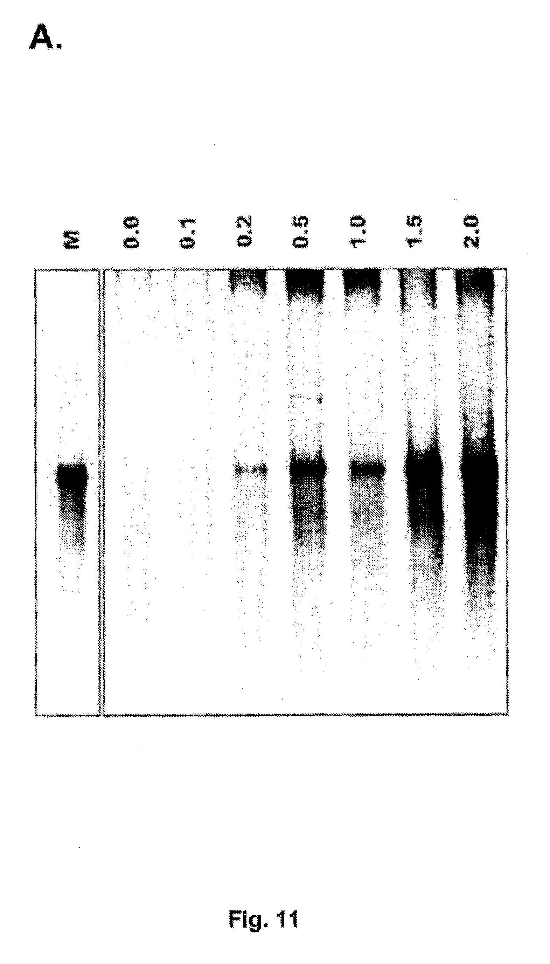

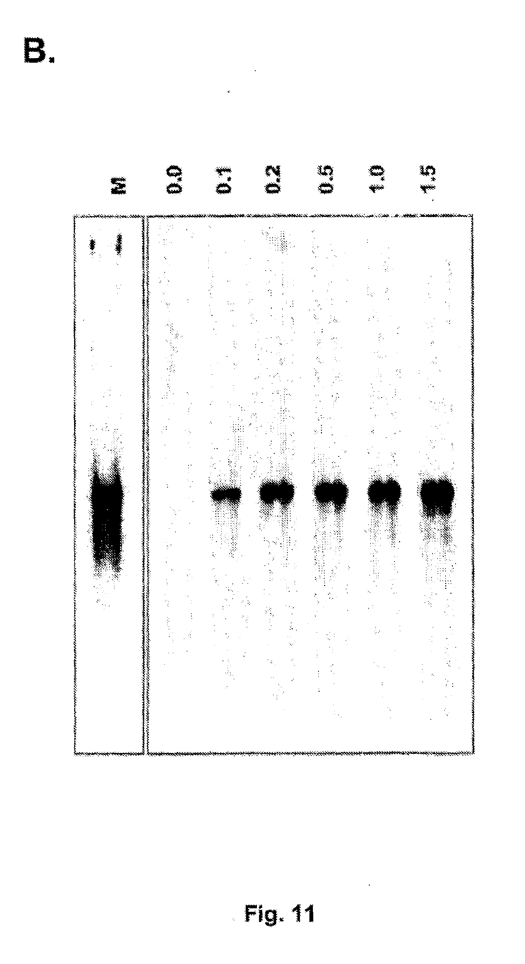

- **Figur 11:**: **Primer-abhängige Replikation von *full-length* subgenomischer polyadenylierter RNA durch Norovirus 3D**^{pol}. Bei allen Reaktionen wurde synthetische subgenomische polyadenylierte RNA als Template eingesetzt. Die Reaktionsprodukte wurden auf Formaldehyd-Agarosegelen analysiert und durch Autoradiographie visualisiert.
**(A)** Die Reaktion wurde in Gegenwart eines oligo(U)₂₀-RNA-Primers wie angegeben (mM) durchgeführt. M, Marker (*in vitro* transkribierte subgenomische polyadenylierte RNA).
**(B)** Die Reaktion wurde in Gegenwart eines RNA-Oligonukleotids durchgeführt, das komplementär zu der dem poly(A)-Schwanz benachbarten Sequenz ist. RNA-Oligonukleotid Primerkonzentration (mM) wie angegeben. M, Marker (*In vitro* transkribierte subgenomische polyadenylierte RNA).

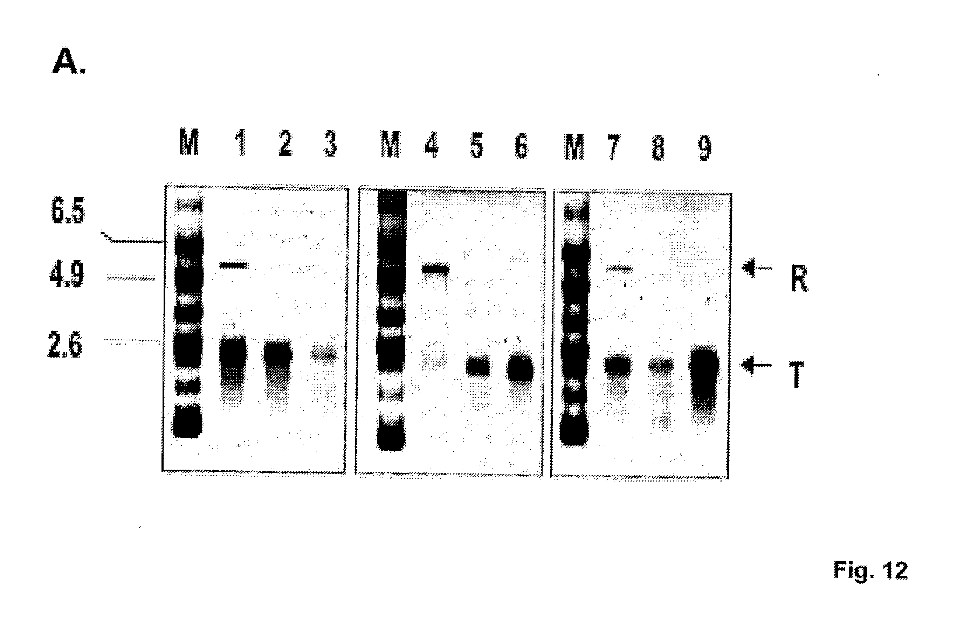

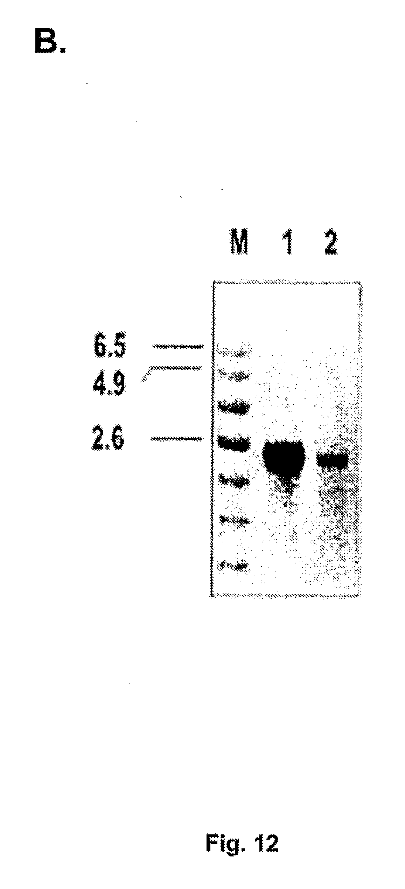

- **Figur 12:**: ***De novo* Initiation der RNA-Synthese an Norovirus anti-subgenomischer RNA.** Bei allen Reaktionen wurde synthetische anti-subgenomische RNA als Template für die Replikationsreakton eingesetzt. Die RNA-Reaktionsprodukte wurden auf nativen Agarosegelen analysiert und nach Ethidiumbromidfärbung durch UV-Transillumination visualisiert.
**(A)** Bahnen 1 bis 3: RNA-Synthese in Gegenwart von Wildtyp 3D^{pol}, mutierter 3D^{pol} bzw. in Abwesenheit von 3D^{pol}. Bahnen 4 bis 6: RNA-Synthese in Gegenwart von Wildtyp 3D^{pol} und einem RNA-Oligonukleotid komplementär zum 3'-Terminus, mutierter 3D^{pol} und einem RNA-Oligonukleotid komplementär zum 3'-Terminus bzw. mit einem RNA-Oligonukleotid komplementär zum 3'-Terminus, aber ohne Norovirus 3D^{pol}. Bahnen 7 bis 9, RNA-Synthese in Gegenwart von Wildtyp 3D^{pol} und einem DNA-Oligonukleotid komplementär zum 3'-Terminus, mutierter 3D^{pol} und einem DNA-Oligonukleotid komplementär zum 3'-Terminus, bzw. mit einem DNA-Oligonukleotid komplementär zum 3'-Terminus, aber ohne Norovirus 3D^{pol}. T, Template-RNA. R, Replikationsprodukt. M, RNA-Molekulargewichtsmarker (Kb).
**(B)** Strangseparationsanalyse der Replikationsprodukte. Die Reaktionsprodukte wurden auf Formaldehyd-Agarosegelen analysiert und nach Ethidiumbromidfärbung durch UV-Transillumitiation visualisiert. Bahnen 1 und 2, Template (anti-subgenomische RNA) bzw. Norovirus 3D^{pol} Replikationsprodukt. M, RNA Molekulargewichtsmarker (Kb).

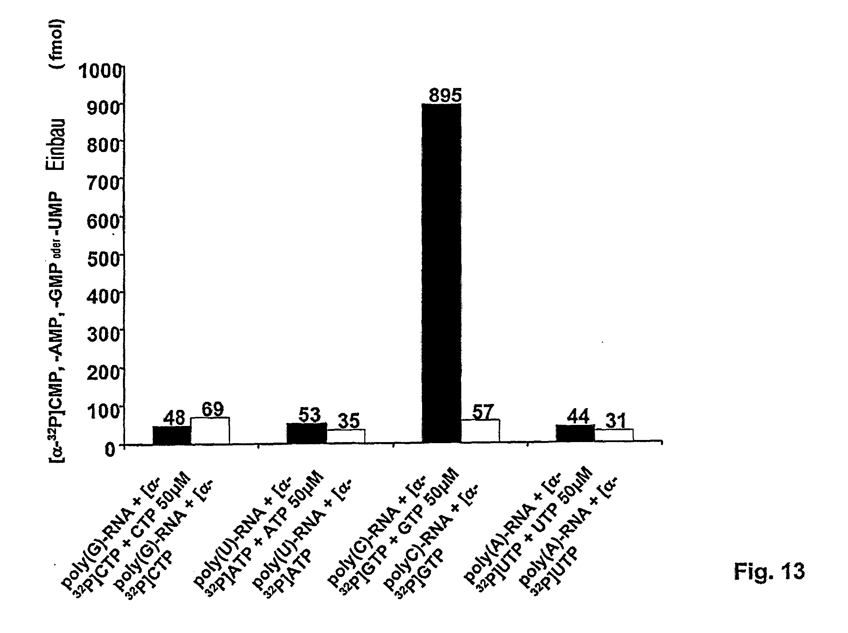

- **Figur 13:**: **Primer-unabhängige *de novo* Initiation der RNA Synthese an homopolymeren Templaten.** Die RNA-Synthese wurde in Anwesenheit (schwarze Balken) oder in Abwesenheit (graue Balken) von kaltem CTP, ATP, GTP bzw. UTP für poly(rG), poly(rU), poly(rC) bzw. poly(rA)-Temptates durchgeführt. Der Einbau von [α-³²P]CMP, [α-³²P]AMP, [α-³²P]GMP, oder [α-³²P]UMP wurde nach TCA-Präzipitation und Sammeln von G/C-Glassfaserfiltern gemessen. Die Einbauwerte sind angegeben.

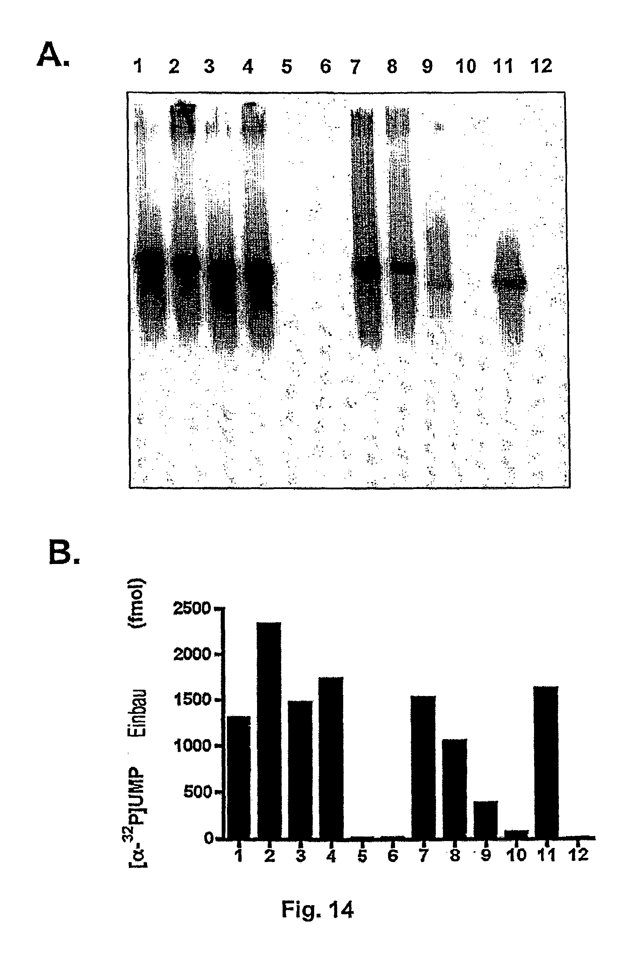

- **Figur 14:**: **Amplifikation viraler und eukaryontischer RNA mit dem Norovirus RNA-Polymerase-Enzym.**
**(A)** Autoradiogramm der inkorporation von [α³²P]UMP. Reaktion 1, Subgenomische Norovirus RNA; Reaktion 2, Subgenomische Norovirus RNA mit einer 60 Nukleotid Deletion am 5'-Ende der RNA; Reaktion 3, Subgenomische Norovirus RNA mit einer 60 Nukleotid Deletion am 3'-Ende der RNA; Reaktion 4, Subgenomische Norovirus RNA mit einer 60 Nukleotid Deletion am 5'-Ende und 3'-Ende der RNA; Reaktion 5 u. 6, negative Kontrolle: Reaktion 7; Subgenomische Astrovirus RNA (Serotyp 1); Reaktion 8; Subgenomische Astrovirus RNA (Serotyp 2); Reaktion 9, eukaryontische RNA des elF4-Gens von *Xenopus Laevi*; Reaktion 10, Subgenomische Norovirus DNA; Reaktion 11; T7-transkriberte RNA des elF4-Gens von *Xenopus Laevi;* Reaktion 12, negative Kontrolle.
**(B)** Quantifizierung der Inkorporation von [α-³²P]UMP, Die Nummerierung der Reaktionen bezieht sich auf die Proben der **Figur 14 (A)****.**

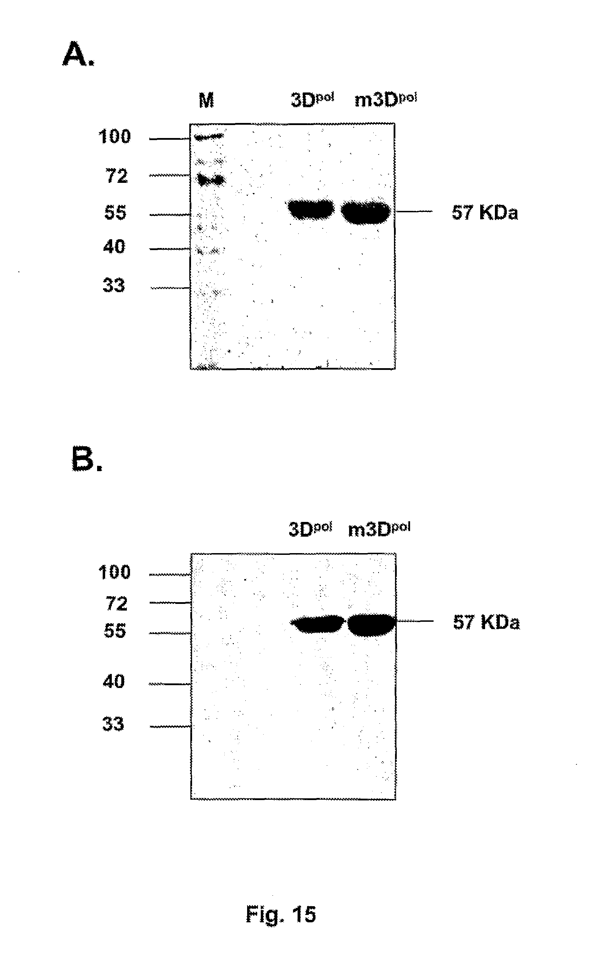

- **Figur 15:**: **Expression und Reinigung von Sapovirus 3D^{pol}** in *E*. *coli.*
**(A)** SDS-PAGE Analyse von gereinigter rekombinanter Sapovirus 3D^{pol} die in *E. coli* exprimiert wurde und einen C-terminalen His-tag trägt. 3D^{pol}, Wildtyp-Sapovirus 3D^{pol}. m3D^{pol}, Sapovirus 3D^{pol} mit einer Mutation in der 'active site' (YGD_{343G}D_{344G}). M, Molekulargewichtsmarker (KDa).
**(B)** Western-Blot Analyse von gereinigter rekombinanter Sapovirus 3D^{pol} unter Verwendung eines Penta-Histidin-Antikörpers (monoklonaler Maus-Antikörper, Qiagen) zur Detektion von Proteinen mit einem C-terminalen His-tag, 3D^{pol}, Wildtyp- Sapovirus 3D^{pol}. m3D^{pol}, Sapovirus 3D^{pol} mit einer Mutation in der 'active site' (YGD_{343G}D_{344G}). M, Molekulargewichtsmarker (KDa).

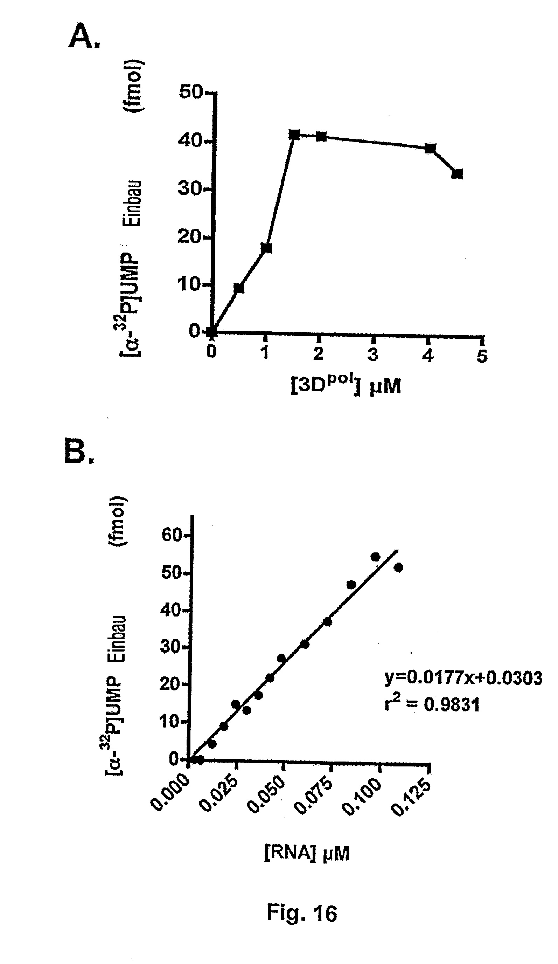

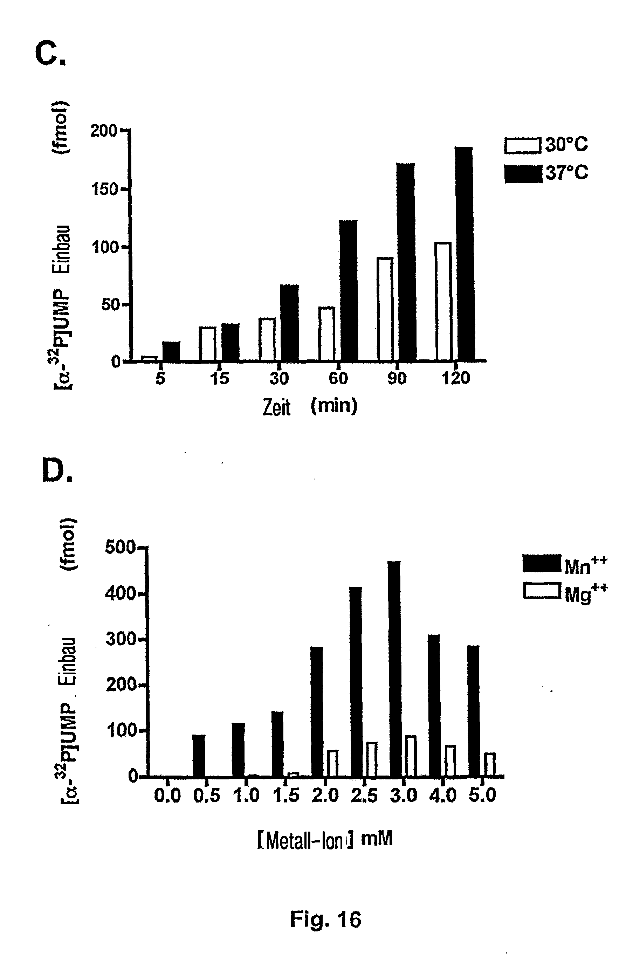

- **Figur 16:**: **Konzentrationsabhängigkeit, Substratabhängigkeit, Temperaturabhängigkeit und Metallionenabhängigkeit der Sapovirus 3D^{pol} Aktivität**. Bei allen Reaktionen wurde subgenomische RNA als Template eingesetzt.
**(A)** Konzentrationsabhängige Aktivität der Sapovirus 3D^{pol}. Die RNA-Synthese wurde in Gegenwart von 0.024 µM RNA und ansteigenden Konzentrationen von Sapovirus 3D^{pol} (0.0 bis 5.0 µM) durchgeführt. Einbau von [α-³²P]UMP wie angegeben. Die Reaktion wurde 2 h bei 37°C durchgeführt.
**(B)** Substratabhängigkeit der Sapovirus 3D^{pol} Aktivität. Die RNA-Synthese wurde in Gegenwart von 3 µM Sapovirus 3D^{pol} und ansteigenden Konzentrationen von subgenomischer RNA als Template (0.00, bis 0.100 µM) Für 2h bei 37°C durchgeführt. Einbau von [α-³²P]UMP wie angegeben.
**(C)** Temperaturabhängige Activität von Sapovirus 3D^{pol}. Die Reaktion wurde bei 30°C oder 37°C durchgeführt und zu den angegebenen Zeiten gestoppt. Bei allen Reaktionen wurden 3 µM Sapovirus 3D^{pol} eingesetzt. Einbau von [α-³²P]UMP wie angegeben.
**(D)** Metallionenabhängigkeit der Sapovirus 3D^{pol}-Aktivität. Die RNA-Synthese wurde in Gegenwart von 3 µM Sapovirus 3D^{pol} mit subgenomischer RNA als Template (0.024 µM) und steigenden Konzentrationen (0.5 bis 5 mM) von MgAcO oder MnCl₂ bei 37°C für 2 h durchgeführt. Einbau von [α-³²P]UMP wie angegeben.

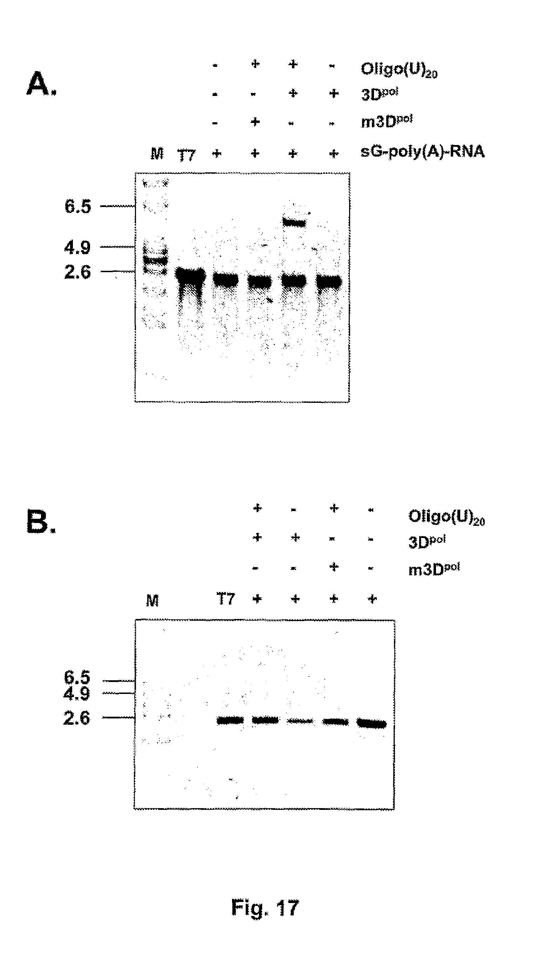

- **Figur 17:**: **RNA-Synthese mit Sapovirus 3D^{pol}.**
**(A)** Die RNA-Synthese in Gegenwart einer synthetischen subgenomischen polyadenylierten RNA (sG-poly(A)-RNA) als Template wurde mit und ohne Zugabe eines 3 µM oligo(U)₂₀ RNA-Primers und in Anwesenheit von Wildtyp Sapovirus 3D^{pol} (3D^{pol}) oder wie angegeben einer in der 'acitve site' mutierten 3D^{pol} (m3D^{pol}, YGD_{343G}D_{344G}) untersucht. Die Reaktionsprodukte wurden auf nicht-denaturierenden Agarosegelen analysiert und nach Ethidiumbromidfärbung mit UV-Transillumination visualisiert. Die sichtbare verbleibende Bande in den Reaktionen, bei denen m3D^{pol} anstelle von Wildtyp 3D^{pol} eingesetzt wurden oder bei denen kein Oligo(U)₂₀ RNA-Primer zugegben wurde, resultiert vom in der Reaktion eingesetzten synthetischen subgenomischen RNA Template. T7, synthetische subgenomische RNA, die mittels *in vitro* Transkription mit T7 hergestellt wurde. M, RNA-Molekulargewichtsmarker.
**(B)** Strangseparationsanalyse des Reaktionsprodukts der in vitro RNA-Synthese mit Sapovirus 3D^{pol}. Das Reaktionsprodukt wurde wie angegeben durch RNA-Synthese mit Sapovirus 3D^{pol} (3D^{pol}) unter Verwendung einer subgenomeischen polyadenylierten RNA (sG-Po)y(A)-RNA) als Template erhalten. Die Reaktionsprodukte wurden auf denaturierenden Formaldehyd-Agarosegelen visualisiert. T7, synthetische subgenomische RNA, die mittels *in vitro* Transkription mit T7 hergestellt wurde. M, RNA-Molekulargewichtsmarker.

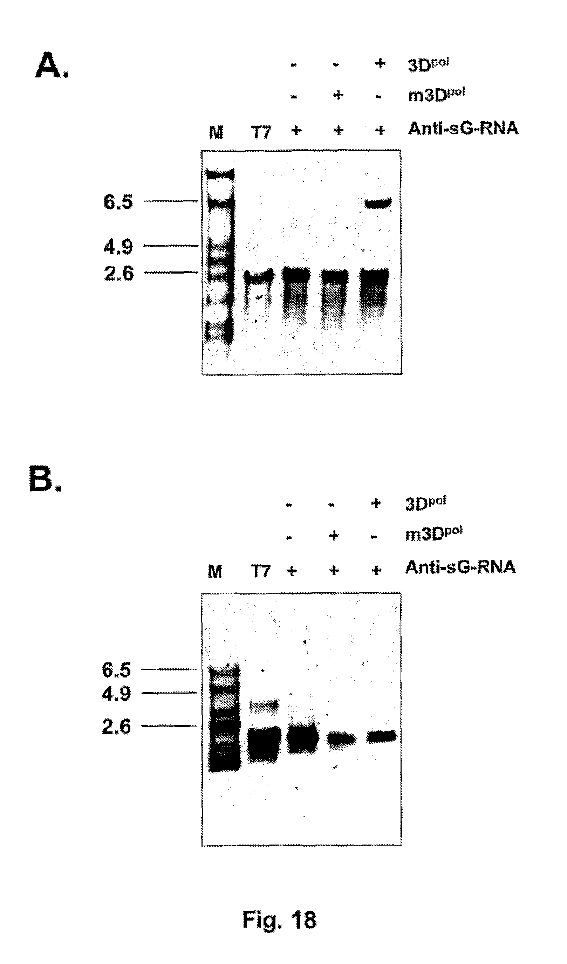

- **Figur 18:**: **De novo Initiation der RNA-Synthese an Sapovirus anti-subgenomischer RNA.**
**(A)** Die RNA-Synthese wurde in Gegenwart einer synthetischen anti-subgenomische RNA (Anti-sG-RNA) als Template und Wildtyp-Sapovirus 3D^{pol} (3D^{pol}) oder einer in der 'active site' mutierten 3D^{pol} (m3D^{pol}, YGD_{343G}D_{344G}), wie angegeben. Die Reaktionsprodukte wurden auf nicht-denaturierenden Agarosegelen analysiert und nach Ethidiumbromidtärbung durch UV Trensilluminafion visualisiert. Die sichtbare verbleibende Bande bei der Reaktion, in der m3D^{pol} anstelle von Wildtyp 3D^{pol} eingesetzt wurde, resultiert aus dem in der Reaktion eingesetzten synthetischen anti-subgenomischen RNA-Template. T7, synthetische subgenomische RNA, die mittels *in vitro* Transkription mit T7 hergestellt wurde. M, RNA-Molekulargewichtsmarker.
**(B)** Strangseparationsanalyse des Reaktionsprodukts der in vitro RNA-synthese mit Sapovirus 3D^{pol}. Das Reaktionsprodukt wurde wie angegeben durch RNA-Synthese mit Sapovirus 3D^{pol} unter Verwendung einer synthetischen anti-subgenomische RNA (Anti-sG-RNA) als Template erhalten. Die Reaktionsprodukte wurden auf denaturierenden Formaldehyd-Agarosegelen visualisiert. T7, synthetische anti-subgenomische RNA, die mittels *in vitro* Transkription mit T7 hergestellt wurde. M, RNA-Molekulargewichtsmarker.

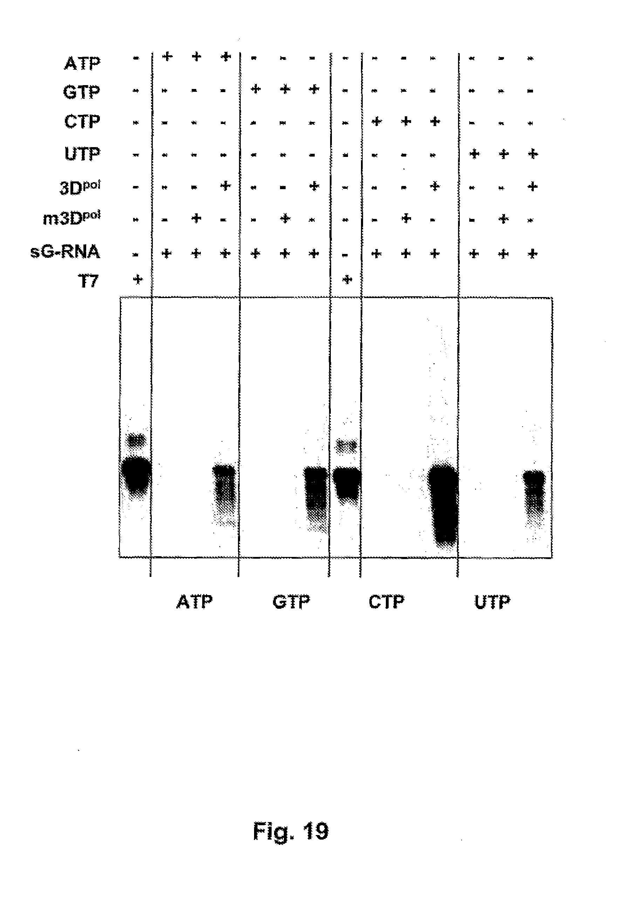

- **Figur 19:**: **Terminale Transferase-Aktivität von Sapovirus 3D^{pol}.**
Die Terminale Transferase-Aktivität wurde anhand einer Reaktion aus 3 µM Sapovirus 3D^{pol} oder einer in der 'active site' mutierten 3D^{pol} (m3D^{pol}) untersucht, die in Gegenwart von subgenomischer RNA (sG-RNA) als Template (0.024 µM) und [α-³²P]ATP, [α-³²P]GTP, [α-³²P]CTP oder [α-³²P]UTP für 2 h bei 37°C (0.024 µM) wie angegeben inkubiert wurde. Die Reaktionsprodukte wurden auf nicht-denaturierenden Agarosegelen analysiert und durch Autoradiographie visualisiert. T7, synthetische subgenomische RNA, die mittels *in vitro* Transkription mit T7 hergestellt wurde.

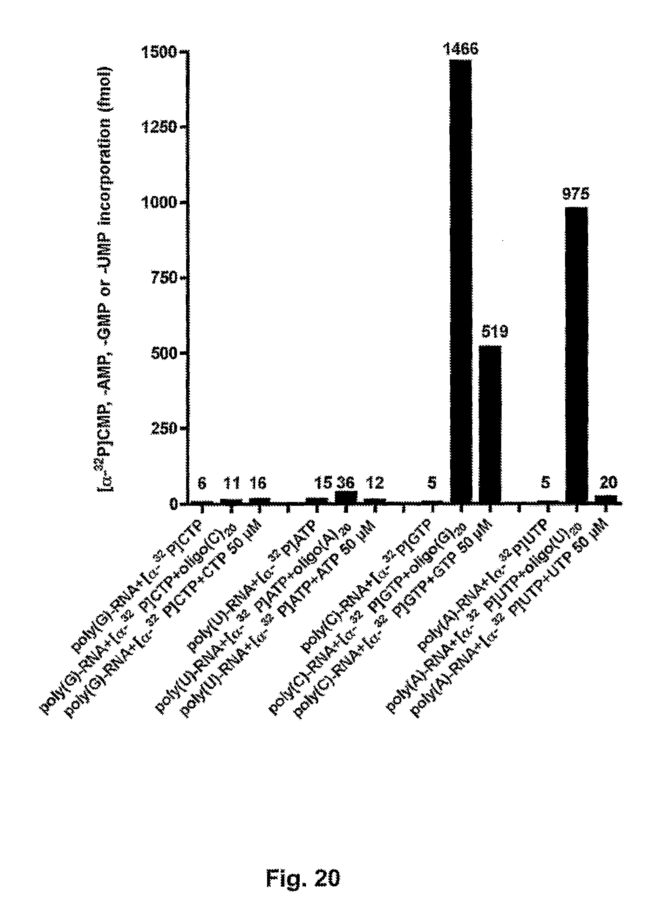

- **Figur 20:**: ***De novo* Initiation der RNA-Synthese durch Sapovirus 3D^{pol} an homopolymeren Templates.**
Die RNA-Synthese wurde durchgeführt, indem poly(G)-RNA, poly(U)-RNA, poly(C)-RNA und poly(A)-RNA Templates mit [α-³²P]CTP, [α-³²P]ATP, [α-³²P]GTP, bzw. [α-³²P]UTP und mit oder ohne 3 µM eines Oligo(C)₂₀, Oligo(A)₂₀, Oligo (G)₂₀ bzw. Oligo(U)₂₀-RNA-Primers inkubiert wurden. Parallel dazu wurde die RNA-Synthese in Abwesenheit eines Oligo(RNA)-Primers, aber in Anwesenheit von 50 µM kaltem CTP, ATP, GTP bzw. UTP für poly(G)-RNA, poly(U)-RNA, poly(C)-RNA bzw. poly(A)-RNA Templates. Einbau von [α-³²P]CMP, [α-³²P]AMP, [α-³²P]GMP, oder [α-³²P]UMP as wie angegeben.

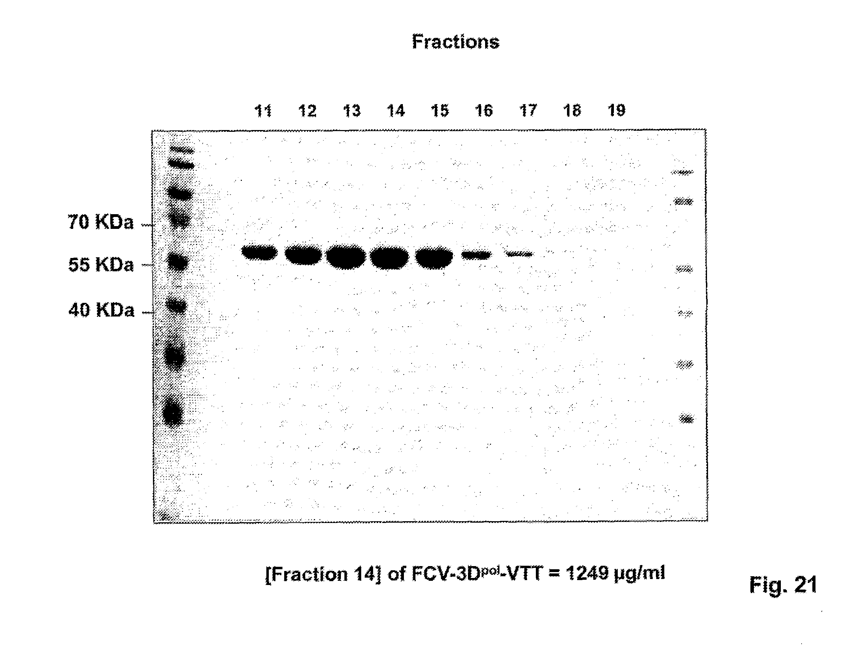

- **Figur 21:**: **Expression und Reinigung vom Vesivirus 3D^{pol} in *E. coli.***
(A) SDS-PAGE Analyse von In *E. coli* exprimierter, gereinigter, rekombinanter Vesivirus 3D^{pol} mit einem C-terminalen His-tag. Gezeigt sind die Fractionen 11 bis 19 nach Affinitätsreinigung an einer Ni-NTA-Säule sowie die Konzentration von Fraktion 14. M, Molekulargewichtsmarker (KDa).

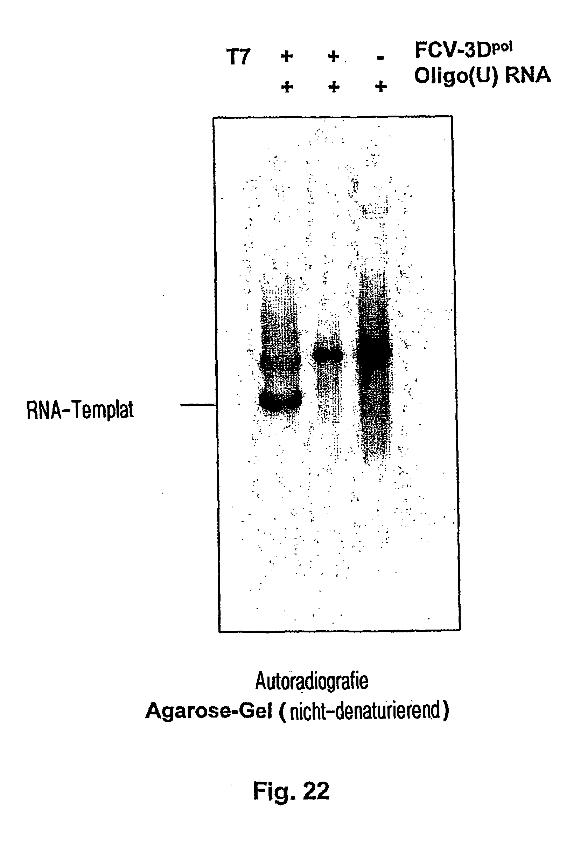

- **Figur 22:**: **RNA-Synthese mit Vesivirus 3D^{pol}.**
(A) Die RNA-Synthese in Gegenwart einer synthetischen subgenomischen polyadenylierten RNA (sG-poly(A)-RNA) als Template wurde mit und ohne Zugabe eines 3 µM oligo(U)₂₀ RNA-Primers und in Anwesenheit von Wildtyp Vesivirus 3D^{pol} (3D^{pol}) untersucht. Die Reaktionsprodukte wurden auf nicht-denaturierenden Agarosegelen analysiert und nach Autoradiographie vizualisiert. T7, synthetische subgenomische RNA, die mittels *in vitro* Transkription mit T7 hergestellt wurde. M, RNA-Molekulargewichtsmarker.

### Ausführungsbelspiel 1

### Verfahren zur Herstellung rekombinanter Norovirus RdRP

Die cDNA der Norovirus-RdRP wurde durch PCR aus dem Norovirus-Klon pUS-Norll (Genbank accession number: AY741811) erhalten. Sie wurde in den pET-28b(+)-Vektor (Novagen) kloniert, der Expressionsvektor wurde sequenziert und in E.coli CL21(DE3)plysS transformiert. Die Zellen wurden bei 37°C in Luria-Bertani-Medium mit Kanamycin (50 mg/L) kultiviert. Bei einer optischen Dichte von 0.6 (OD600) wurde die Proteinexpression durch den Zusatz von lsopropyl-β-D-thiogalactopyranosid (IPTG) mit einer Endkonzentration von 1 mM induziert. Die Kulturen wurden dann bei 25°C über Nacht inkubiert. Die aus 250 ml Kultur erhaltenen Zellpellets wurden einmal in 4 ml phosphate buffered saline (PBS) und 1 % Triton X 100 (Sigma) gewaschen. Die Zellen wurden bei 37°C für 15 Minuten mit DNase (10 U/ml) behandelt, auf Eis sonifiziert und in 40 ml Bindungspuffer (20 mM Tris/HCl, pH 7.9, 500 mM NaCl, 5 mM Imidazol) resuspendiert. Nach der Zentrifugation bei 4300 rpm bei 4 °C für 40 Minuten wurde das geklärte Lysat erhalten. Die mit einem His6-tag versehene Norovirus-RdRP wurde an eine Ni-Nitrilotriacetessigsäure (NTA)-Sepharosematrix (Novagen) gebunden, die mit dem Bindungspuffer prääquilibriert worden war. Das gebundene Protein wurde mit dem Bindungspuffer, der 60 mM Imidazol enthielt, gewaschen und mit Bindungspuffer, der 1 M Imidazol enthielt, eluiert. Das eluierte Protein wurde gegen Puffer A dialysiert (25 mM Tris-hCl, pH 8.0, 1 mM β-Mercaptoethanol, 100 mM NaCl, 5 mM MgCl2, 10 % Glycerin, 0.1 % Triton X). Die Proteinkonzentration wurde mit dem BCA proteinassay Kit (Pierce) auf der Grundlage der Biuret-Reaktion bestimmt. Das Enzym wurde in einem Endvolumen von 50 % Glycerin resuspendiert und bei -20°C aufbewahrt.

### Ausführungsbeispiel 2

### Sequenzunabhängige RNA-Amplifikation mit Norovirus-RdRP

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-inhibitor (RNAsin, Promega), je 0.4 mM von ATP, CTP, GTP, UTP, 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufferten Agarosegelen sichtbar gemacht. Formamid-Agarosegele können ebenfalls verwendet werden.

### Ausführungsbeispiel 3

### Sequenzabhängige RNA-Amplifikation mit Norovirus-RdRP mit einem genspezifischen RNA-Primer

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-Inhibitor (RNAsin, Promega), je 0.4 mM von ATP, CTP, GTP, UTP, 0.1 bis 1 µM genspezifischer RNA-Primer, 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufferten Agarosegelen sichtbar gemacht. Formamid-Agarosegele oder Harnstoff/Polyacrylamidgele können ebenfalls verwendet werden.

### Ausführungsbeispiel 4

### Amplifikation der zellulären mRNA mit Norovirus-RdRP mit einem poly-U-Primer

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-Inhibitor (RNAsin, Promega), je 0.4 mM von ATP, CTP, GTP UTP, 0.1 bis 1 µM poly-(U)₂₀-Primer, 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufferten Agarosegelen sichtbar gemacht. Formamid-Agarosegele können ebenfalls verwendet werden.

### Ausführungsbeispiel 5

### Herstellung von siRNA mit Norovirus-RdRP

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-Inhibitor (RNAsin, Promega), je 0.4 mM von ATP, CTP, GTP, UTP, 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufferten 10 %igen Polyacrylamidgelen sichtbar gemacht. Formamid-Agarosegele können ebenfalls verwendet werden. Alternativ kann armored-RNA verwendet werden.

### Ausführungsbeispiel 6

### Labelling von RNA mit Norovirus-RdRP

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-Inhibitor (RNAsin, Promega), je 0.4 mM von ATP, oder CTP, oder GTP, oder UTP, 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufierten 10 %igen Polyacrylamidgelen sichtbar gemacht. Formamid-Agarosegele können ebenfalls verwendet werden. Alternativ kann *armored-RNA* verwendet werden.

### Ausführungsbeispiel 6

### RNA-Synthese von poly(C)-poly(G) RNA-Hybrid mit Norovirus-RdRP

Die Reaktionsmischung (50 µl) besteht aus 0.5 bis 1 µg poly(C)-RNA-Matrize, oder eine RNA mit poly(C)-RNA am 3'-Ende, 10 µl des Reaktionspuffer (250 mM HEPES, pH 8.0, 15 mM Magnesiumacetat, 20 mM DTT), 50 U RNase-Inhibitor (RNAsin, Promega), 50 µM von GTP, und 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Emidiumbromid-Färbung durch UV-Ttansillumination auf TBE-gepufferten 10 %igen Polyacrylamidgelen sichtbar gemacht. Formamid-Agarosegele können ebenfalls verwendet werden. Alternativ kann *armored*-RNA verwendet werden.

Weiterhin umfasst die vorliegende Erfindung eine RNA-abhängige RNA-Polymerase zur Amplifikation und/oder Markierung von RNA, wobei die RNA-abhängige RNA-Polymerase eine "Rechte-Hand-Konformation" aufweist und die Aminosäuresequenz der RNA-abhängigen RNA-Polymerase die folgenden Sequenzabschnitte umfasst: a. XXDYS, b. GXPSG, c. YGDD, d. XXYGL, e. XXXXFLXRXX mit der Bedeutung: D: Aspartat, Y: Tyrosin, S: Serin, G: Glycin, P: Prolin, L: Leucin, F: Phenylalanin, R: Arginin, X: eine beliebige Aminosäure.

Eine bevorzugte Ausführungsform der RNA-abhängigen RNA-Polymerase ist eine RNA-abhängige RNA-Polymerase eines Virus aus der Familie der *Caliciviridae.*

Bei einer weiteren bevorzugten Ausführungsform ist die RNA-abhängige RNA-Polymerase eine RNA-abhängige RNA-Polymerase eines humanen und/oder nicht-humanen pathogenen Virus der Familie der *Caliciviridae.*

Bei einer weiteren bevorzugten Ausführungsform ist die RNA-abhängige RNA-Polymerase eine RNA-abhängige RNA-Polymerase eines Norovirus, Sapovirus, Vesivirus oder eines Lagovirus.

Bei einer weiteren bevorzugten Ausführungsform ist die RNA-abhängige RNA-Polymerase die RNA-abhängige RNA-Polymerase eines Norovirus-Stammes wie z.B. des Norovirus-Stammes HuCV/NL/Dresden174/1997/GE oder eines eines SapovirusStammes wie z.B. des Sapovirus-Stammes pJG-SapO1, oder eines Vesivirus-Stammes wie z.B. des Vesivirus-Stammes FCV/Dresden/2006/GE.

Bei einer weiteren bevorzugten Ausführungsform ist die RNA-abhängige RNA-Polymerase ein Protein gemäß SEQ ID NO 1, SEQ ID NO 2, SEQ ID NO 3, SEQ ID NO 4, SEQ ID NO 5 oder SEQ ID NO 6.

Die vorliegende Erfindung umfasst weiterhin ein Verfahren zur Amplifikation von RNA mittels einer RNA-abhängigen RNA-Polymerase mit den Schritten a. Anlagerung der RNA-abhängigen RNA-Polymerase an die RNA-Matrize in der Anwesenheit oder Abwesenheit eines Oligonukleotid-Primers, b. Umschreiben der RNA-Matrize in antisense-RNA durch die RNA-abhängige RNA-Polymerase c. Trennung des RNA/antisense-RNA-Duplexes in einzelne RNA-Stränge.

Bei einer bevorzugten Ausführungsform des Verfahrens zur Amplifikation von RNA erfolgt die Trennung des RNA/antisense-RNA-Duplexes in einzelne RNA-Stränge durch Hitzedenaturierung, durch chemische Denaturierung und/oder enzymatisch.

Bei einer weiteren bevorzugten Ausführungsform erfolgt die enzymatische Trennung des RNA/antisense-RNA-Duplexes in einzelne RNA-Stränge durch ein Enzym mit der Fähigkeit, doppelsträngige RNA in einzelsträngige RNA aufzutrennen.

Weiterhin umfasst die vorliegende Erfindung ein Verfahren zur primerabhängigen Amplifikation von RNA bei dem mindestens ein Primer, der mit einem Abschnitt der RNA-Matrize hybridisiert, eingesetzt wird und nach der Anlagerung der RNA-abhängigen RNA-Polymerase der Primer durch die RNA-abhängige RNA-Polymerase entsprechend der Sequenz der RNA-Matrize verlängert wird.

Bei einer bevorzugten Ausführungsform dieses Verfahrens wird als Primer ein heteropolymeres oder homopolymeres RNA-Primer oder DNA-Primer eingesetzt.

Bei einer weiteren bevorzugten Ausführungsform wird als Primer ein poly-U-RNA-, poly-A-RNA-, poly-C-RNA- oder poly-G-RNA-Primer oder jedes beliebiges hompolymeres Oligo-RNA-Primer eingesetzt.

Bei einer weiteren Ausführungsform des Verfahrens zur primerunabhängigen Amplifikation von poly(C)-RNA wird kein Primer, der mit einem Abschnitt der RNA-Matrize hybridisiert, eingesetzt, GTP als einziges Nukleotid verwendet und durch die RNA-abhängige RNA-Polymerase entsprechend der Sequenz der RNA-Matrize verlängert.

Die vorliegende Erfindung umfasst weiterhin ein Verfahren zur Markierung von RNA mittels einer RNA-abhängigen RNA-Polymerase, mit den Schritten: a. Anlagerung der RNA-abhängigen RNA-Polymerase an die zu markierende RNA, b. Anhängen mindestens eines Nukleotids an das 3'-Ende der zu markierenden RNA.

Die vorliegende Erfindung betrifft ebenfalls die Verwendung einer RNA-abhängigen RNA-Polymerase zur Amplifikation und/oder Markierung von RNA.

Weiterhin umfasst die vorliegende Erfindung ein Kit zur Amplifikation von RNA, mindestens bestehend aus: a. einer RNA-abhängigen RNA-Polymerase nach einem der Ansprüche 1 bis 6, b. einem geeigneten Reaktions-Puffer, c. NTPs, d. ggf. RNase-Inhibitor, e. ggf. Stoplösung.

Ein weiterer Gegenstand der Erfindung ist ein Kit zur Amplifikation von RNA, mindestens bestehend aus a. einer RNA-abhängigen RNA-Polymerase nach einem der Ansprüche 1 bis 6, b. einem geeigneten Reaktions-Puffer, c. NTPs, d. ggf. RNase-Inhibitor, e. ggf. Stoplösung, f. einer Helicase.

Ein weiterer Gegenstand der Erfindung ist ein Kit zur primerabhängigen Amplifikation von RNA, mindestens bestehend aus: a. einer RNA-abhängigen RNA-Polymerase nach einem der Ansprüche 1 bis 6, b. einem geeigneten Reaktions-Puffer, c. NTPs, d. ggf. RNase-Inhibitor, e. ggf. Stoplösung, f. Primer.

Die vorliegende Erfindung umfasst zudem ein Kit zur primerunabhängigen Amplifikation von RNA, mindestens bestehend aus: a. einer RNA-abhängigen RNA-Polymerase nach einem der Ansprüche 1 bis 6, b. einem geeigneten Reaktions-Puffer, c. NTPs, d. ggf. RNase-Inhibitor, e. ggf. Stoplösung.

Weiterhin umfasst die vorliegende Erfindung ein Kit zur Markierung von RNA, mindestens bestehend aus a. einer RNA-abhängigen RNA-Polymerase nach einem der Ansprüche 1 bis 6, b. einem geeigneten Reaktions-Puffer, c. CTP oder UTP oder ATP oder GTP oder jeden beliebigen Nukleotid, d. ggf. RNase-Inhibitor, e. ggf. Stoplösung.

## Patentansprüche

1. RNA-abhängige RNA-Polymerase mit einer Aminosäuresequenz gemäß SEQ ID NO: 2.

2. RNA-abhängige RNA-Polymerase nach Anspruch 1, die am N- oder C-Terminus mit einer Sequenz fusioniert ist, welche die Aufreinigung des Fusionsproteins nach Expression in einem Wirtsorganismus erleichtert.

3. RNA-abhängige RNA-Polymerase nach Anspruch 2, wobei die Sequenz ein Histidin-Marker ist.

4. RNA-abhängige RNA-Polymerase nach Anspruch 3, die eine Aminosäuresequenz gemäß SEQ ID NO: 5 aufweist.

5. Nukleinsäure, die für eine RNA-abhängige RNA-Polymerase nach einem der vorhergehenden Ansprüche kodiert.

6. Expressionsvektor, der eine Nukleinsäure nach Anspruch 5 enthält.

7. Wirtsorganismus, der einen Expressionsvektor nach Anspruch 6 enthält.

8. Verfahren zur Herstellung der RNA-abhängigen RNA-Polymerase nach einem der Ansprüche 1 bis 4 mit den Schritten
i. Klonierung der die RNA-abhängige RNA-Polymerase kodierenden Nukleinsäure in einen Expressionsvektor und
ii. Einbringen des Expressionsvektors in einen Wirtsorganismus.

9. Kit zur Amplifikation von RNA, mindestens bestehend aus
- einer RNA-abhängigen RNA-Polymerase nach einem der Ansprüche 1 bis 4
- einem geeigneten Reaktionspuffer
- Ribonukleotide ATP, UTP, GTP und CTP;
- ggf. Stoplösung;
- ggf. RNAse-Inhibitor.

10. Kit nach Anspruch 9, das mindestens einen poly-U-RNA-Primer enthält.

11. Kit nach Anspruch 9 oder 10, das mindestens einen Primer enthält, der spezifisch mit einem Abschnitt viraler RNA hybridisiert.

12. Kit zur Markierung von RNA, mindestens bestehend aus
- einer RNA-abhängigen RNA Polymerase nach einem der Ansprüche 1 bis 4;
- einem geeigneten Reaktionspuffer;
- ATP, GTP, UTP oder CTP;
- ggf. Stoplösung;
- ggf. RNAse-Inhibitor.
